(19) Europäisches Patentamt
European Patent Office
Office européen des brevets

(11) Publication number: 0 097 949 B1

# (12) EUROPEAN PATENT SPECIFICATION

(45) Date of publication of patent specification: 22.03.89

(51) Int. Cl.$^4$: C 12 N 9/06, C 12 Q 1/26, C 12 M 1/40 // C12R1/465

(21) Application number: 83106258.3

(22) Date of filing: 27.06.83

## (54) L-glutamic acid oxidase, its production, and its use.

(30) Priority: 29.06.82 JP 112271/82
21.08.82 JP 145346/82
23.08.82 JP 146707/82

(43) Date of publication of application:
11.01.84 Bulletin 84/02

(45) Publication of the grant of the patent:
22.03.89 Bulletin 89/12

(84) Designated Contracting States:
AT BE CH DE FR GB IT LI NL SE

(56) References cited:
FR-A-2 522 680

ANNUAL MEETING OF THE JAPANESE BIOCHEMICAL SOCIETY, OSAKA (JP), October 12, 1982, & Agr.Biol.Chem., vol. 47, no. 6, June 1983, pp. 1323-1328, H. KUSAKABE et al.: "Purification and properties of a new enzyme, L-glutamate oxidase, from streptomyces sp. X-119-6, grown on wheat bran"

(73) Proprietor: Yamasa Shoyu Kabushiki Kaisha
10-1, Araoi-Cho 2-Chome
Choshi-Shi Chiba-Ken 288 (JP)

(72) Inventor: Kusakabe, Hitoshi
2-2, Sakae-cho, 2-chome
Choshi-shi Chiba-ken (JP)
Inventor: Midorikawa, Yuichiro
5201-7, Ashikajima-cho
Choshi-shi Chiba-ken (JP)
Inventor: Yamauchi, Hiroshi
2-1, Araoi-cho 2-chome
Choshi-shi Chiba-ken (JP)

(74) Representative: Dr. Elisabeth Jung Dr. Jürgen Schirdewahn Dipl.-Ing. Claus Gernhardt
P.O. Box 40 14 68 Clemensstrasse 30
D-8000 München 40 (DE)

(56) References cited:
AGRIC. BIOL. CHEM., vol. 47, no. 1, January 1983, pp. 179-182, H. KUSAKABE et al.: "Occurence of a new enzyme, L-Glutamate oxidase in a wheat bran culture extract of streptomyces sp. X-119-6"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 097 949 B1

Courier Press, Leamington Spa, England.

(56) References cited:

**CHEM. PHARM. BULL., vol. 31, no. 4, April 1983, pp. 1307-1314, T. KAMEI et al.: "L-Glutamate oxidase from streptomyces violasces. I. Production, isolation and some properties"**

**CHEMICAL ABSTRACTS vol. 82, 1975, p. 187, COLUMBUS OHIO (US), abstract 82194w, JURTSHUK, P. et al.: "Nonpyridine nucleotide dependent L-(+)-glutamate oxidoreductase in Azoto-bacter vinelandii"**

**CHEMICAL ABSTRACTS, vol. 97, 1982, p. 449, COLUMBUS OHIO (US), abstract 37 526e**

**Description**

Background of the invention

Field of the art

This invention relates to a novel L-glutamic acid oxidase and its use, particularly to its use for analysis of L-glutamic acid.

More specifically, the present invention relates to an L-glutamic acid oxidase which exhibits a strong affinity and a high substrate specificity for L-glutamic acid but has substantially no action on L-glutamine, L-aspartic acid and L-histidine and yet has a high pH-stability and a high thermal stability, an analytical method for assay of L-glutamic acid in a sample to be analyzed by the use of this enzyme, a reagent for analysis to practice the analytical method, a kit for analysis comprising said reagent, and a biosensor employing said enzyme.

Prior art

In the prior art, as methods for analysis of L-glutamic acid, the chromatographic method, the microbiological quantitative determination method, the electrophoresis method, and the enzymatic method have been known. Among these methods, the chromatographic method and the enzymatic method are most generally used.

As the chromatographic method, the method in which an amino acid autoanalyzer is employed is generally practiced. This method, while it is an excellent method of high precision as well as high reliability, uses an expensive apparatus and may also involve a problem in that deproteinization may be sometimes required depending on the sample to make the method for sample preparation complicated.

The enzymatic methods known in the art are (1) the method in which an L-glutamic acid decaboxylase is used and (2) the method in which an L-glutamic acid dehydrogenase is used. However, these methods employing these enzymes have the following problems.

In the method (1) wherein an L-glutamic acid decarboxylase is employed, measurement of the amount of L-glutamic acid is carried out by detection of carbon dioxide which is the reaction product, for which there is generally employed (a) the method using a Warburg's manometer or (b) the method using an autoanalyzer.

The method (a) using a Warburg's manometer affords high precision, but it requires considerable skill of an expert, takes a long time for measurement, and also has a low sample-processing capacity. On the other hand, in the method (b) using an autoanalyzer, carbon dioxide is absorbed in a sodium carbonate solution of phenolphthalein, and the amount of carbon dioxide generated is measured by the degree of color reduction. Therefore preliminary treatments such as degassing of carbon dioxide and oxygen from an enzyme solution and a buffer are required under cooling, and also gas-liquid separation is necessary after the reaction, whereby the apparatus becomes disadvantageously complicated.

According to this enzymatic method, as a L-glutamic acid decarboxylase, an enzyme obtained from a pumpkin of *E. coli* is generally used. The enzyme from a pumpkin has a urease activity, and therefore when a sample containing urea is to be measured, measurement errors due to carbon dioxide from the urea may occur. The activity of this enzyme is also inhibited by an organic acid such as acetic acid, etc., and therefore, when a sample contains a large amount of organic acids, the enzymatic activity may be inhibited by the acids contained in the sample to provide inaccurate results. On the other hand, an enzyme from *E. coli* exhibits activities for L-arginine and L-glutamine and therefore an accurate result cannot be obtained for a sample to be measured containing such a large amount of these amino acids as to have a substantial influence on the analysis of L-glutamic acid. Also, storage stability of the enzyme *per se* is not good.

The L-glutamic acid dehydrogenase used in the method (2) catalyzes the reaction in which L-glutamic acid is deaminated in the presence of NAD (oxidized form of nicotinamide adenine dinucleotide) to form α-ketoglutaric acid and ammonia accompanied with the formation of NADH (reduced form of nicotinamide adenine dinucleotide). In the method using this enzyme, measurement of the reaction is conducted by detection of the amount of NADH formed through the increase in absorbance at 340 nm. However, the equilibrium in this enzymatic reaction is more inclined toward formation of L-glutamic acid, and, for analysis of L-glutamic acid by the use of this enzyme, the equilibrium of this reaction must be shifted toward formation of α-ketoglutaric acid, and various contrivances are required therefor. For this purpose, a trapping agent for α-ketoglutaric acid is generally added into the reaction system, but such an agent may sometimes interfere with the reaction unless its concentration is strictly controlled. Further, the NAD concentration must also be controlled strictly. In the case of measuring a sample containing a substance exhibiting absorption at the wavelength to be measured such as soy sauce, the value obtained must be corrected by using the value obtained in a blank test. Also, a lactic acid dehydrogenase may sometimes exist in the enzyme employed, and the influence of such an enzyme activity must also be taken into consideration.

Recently, an L-amino acid oxidase having a substrate specificity for L-glutamic acid has been found to be produced by cultivation of a microorganism belonging to the genus *Streptomyces* (hereinafter sometimes abbreviated as "*S.*"), more specifically *Streptomyces violascens* (See Japanese Patent Laid-Open Publication No. 43685/1982). The physicochemical properties of the glutamic acid oxidase

(hereinafter sometimes abbreviated as "known enzyme") as a protein have not yet been clarified, but the known enzyme is described to have enzymological properties as follows.

(1) Substrate specificity
When the velocity of enzymatic reaction for L-gluatmic acid is given as 100, the known enzyme has a relative activity of 8.4 for L-glutamine and of 6.8 for L-histidine, exhibiting substantially no activity for other amino acids.

(2) Optimum pH
pH 5—6

(3) pH stability
Stable in the range of pH 3.5—6.5 (37°C, maintained for one hour)

(4) Temperature stability
Stable up to 50°C (maintained for 10 minutes)

(5) Influence of inhibitors
Substantially completely inhibited by mercury ions, copper ions and diethyldithiocarbamate.

The specification of the above Laid-Open Publication states that a liquid culture of the aforesaid microorganism is preferable for production of the known enzyme.

In the utilization of the known enzyme for analysis of L-glutamic acid, various problems are involved. Specifically, although the known enzyme has a higher substrate specificity for L-glutamic acid as compared with other L-amino acid oxidases known in the art, it still exhibits clear activities for other amino acids as mentioned above, and therefore it cannot be used for specific quantitative determination of L-glutamic acid in the presence of these amino acids. Also, the known enzyme does not have a high pH stability and a high heat stability, and it cannot be considered to always have a good storage stability and usage stability as a reagent for analysis. Further, when copper ions exist in a sample to be analyzed, the activity of the known enzyme is markedly inhibited, whereby analysis may be considered to become difficult. Furthermore, the pH of reaction solutions employed in various clinical biochemical diagnostic analysis, especially in analysis of the activity of enzymes in blood is usually around neutral, while the known enzyme will completely lose its activity at a pH of 7.5 when treated at 37°C for one hour. For this reason, it may be difficult to use the known enzyme in analysis in around the neutral pH range.

One other view point in the technique of analyzing L-glutamic acid is of importance: basically the method using a biosensor is known.

In the prior art, as biosensors for analysis of L-glutamic acid, (1) an enzyme electrode using L-glutamic acid dehydrogenase as the receptor portion of L-glutamic acid and a cation electrode as the transducer portion [Anal. Chim. Acta, *56*, 333 (1971)] and (2) a microorganism electrode using the lyophilized cell of *E. coli*, which exhibits L-glutamic acid decarboxylase activity, as the receptor portion of L-glutamic acid and a carbon dioxide electrode as the transducer portion [Anal. Chim. Acta, *116*, 61 (1980)] have been known.

The enzyme electrode of (1) is not practical since it has an extremely poor stability of the enzyme (only for 2 days) and also is susceptible to the influence of cations coexisting in the same measurement system such as sodium ion and potassium ion because of the use of a cation electrode. The microorganism electrode of (2) has an excellent stability (for 3 weeks, 1,500 times or more) because an immobilized microorganism cell is employed. However, carbon dioxide is generated through the aspiration action of the cell under aerobic conditions to exert an influence on the measurement. For removal of such an influence, it is necessary to inhibit the aspiration action of the cell and also to remove carbon dioxide contained in the sample by, for example, blowing of nitrogen gas into the reaction mixture. Also, since the microorganism electrode does not have a high substrate specificity as tabulated below, it can be utilized only for rough measurements as in a process control of fermentation.

Substrate specificity of microorganism electrode

| Amino acid | Relative sensitivity ratio |
|---|---|
| Glutamic acid | 100 |
| Glutamine | 108 (11*) |
| Alanine | 0.5 |
| Arginine | 0.6 |
| Aspartic acid | 1.0 |
| Cystine | 0.4 |
| Glycine | 0.4 |
| Tryptophan | 0.4 |

*: Acetone treated cell was employed.

An enzyme electrode using an L-amino acid oxidase as the receptor portion is also known [Anal. Chem., *47,* 1359 (1975)]. However, the L-amino acid oxidase of the prior art used in such an enzyme electrode acts on L-glutamic acid to a very small extent, and no specific analysis of L-glutamic acid has heretofore been possible by the use of the above enzyme electrode.

There is no disclosure in the above Laid-Open Publication as to whether or not the aforesaid known enzyme can be utilized for a biosensor for specific analysis of L-glutamic acid. Even if the known enzyme can be utilized as the receptor portion of a biosensor, it is possible for various problems to arise. That is, the known enzyme, while it has a relatively higher substrate specificity for L-glutamic acid as compared wih L-amino acid oxidases known in the prior art, still exhibits clear activities for other amino acids as mentioned above, and therefore it cannot be utilized as the biosensor for specific analysis of L-glutamic acid in the co-presence of these amino acids. Also, the known enzyme does not have a high stability, and it cannot be considered to necessarily have a good storage stability and usage stability when utilized as the receptor portion of the biosensor. Further, since the known enzyme is extremely unstable in the pH range above 7 it may be difficult to use the known enzyme biosensor in around the neutral pH range in which various biochemical analyses in the clinical field should be carried out. Furthermore, when copper ions are present in a sample to be analyzed, the activity of the known enzyme may be considered to be markedly inhibited by such ions, whereby analysis becomes difficult.

As described above, there has been in the prior art neither a biosensor for analysis of L-glutamic acid utilizing an oxidase as the receptor portion for specific recognition of L-glutamic acid nor an L-amino acid oxidase capable of accomplishing sufficiently such an object.

Summary of the invention

We have made investigations concerning enzymes which can oxidatively deaminate L-amino acids among the cultured products of microorganisms and as a result have found that there exists a specific novel L-amino acid oxidase having an extremely high substrate specificity for L-glutamic acid in the cultured product of an actinomycete newly isolated from a soil sample. We have isolated and purified the enzyme of the present invention as a single enzyme protein from such a cultured product of the microorganism to accomplish the present invention.

The present invengion provides an oxidase which is an L-amino acid oxidase having the activity of oxidative deamination of the α-amino group of L-glutamic acid in the presence of water and oxygen to form α-ketoglutaric acid, ammonia and hydrogen peroxide, having a very high substrate specificity for L-glutamic acid substantially without acting on L-glutamine, L-aspartic acid and L-histidine and having a high pH-stability and a high temperature stability which is characterized in that

a) it is stable in the pH range from pH 5.5 to pH 10.5 under the maintenance conditions of 37°C and 60 minutes;

b) has its optimum activity in the pH range from 7 to 8.5;

c) is not inactivated by cupric chloride in a concentration of 1.0 mM at pH 7.4 (see Table 2);

d) contains 2 mols of flavin adenine dinucleotide per mol of the enzyme as a coenzyme;

e) has a molecular weight of 135,000±10,000 as estimated by the gel filtration method;

f) is obtainable by culturing a microorganism of the species Streptomyces X-119-6 (FERM P-6560).

The enzyme of the present invention acts specifically on L-glutamic acid substantially without action on other amino acids, and therefore it is suitable for quantitative determination of L-glutamic acid in a system containing many kinds of amino acids. Its specificity for L-glutamic acid is so high that no pre-treatment whatsoever of the sample such as fractionation of amino acids in the sample is required in carrying out the analysis. For example, it can be used for simple, rapid and specific measurement of glutamic acid content in foods containing many kinds of amino acids such as soy sauce, extracts, liquid seasonings, etc., said glutamic acid content being an important index in quality evaluation, for the process control or the process analysis in such fields as glutamic acid fermentation and production of soy sauce, and for screening of glutamic acid producing microorganisms. Also, since activity assays of enzymes forming glutamic acid as the product such as glutaminase, glutamic acid-oxaloacetic acid transaminase (GOT), glutamic acid-pyruvic acid transaminase (GPT), and γ-glutamyl transpeptidase (γ-GTP) can easily be done by the use of the enzyme of the present invention, this enzyme is useful in clinical diagnosis or in the field of biochemistry.

The enzyme of the present invention has also an advantage in the assay of its enzyme activity since its enzymatic reaction is an oxidase reaction most widely practiced in clinical diagnosis or food analysis.

Further, the enzyme of the present invention has a high stability when compared with enzymes for analysis in general including known enzymes, and therefore it can be utilized as an enzyme electrode for a glutamic acid sensor and expected to be utilized as a labelling enzyme in the enzyme immunoassay (see Japanese Patent Laid-Open Publication No. 37261/1982), and further therefore the reagent for analysis of the present invention is stable in storage and use, resulting in general applicability and economical advantage.

The present invention also provides a method for analysis of L-glutamic acid, which comprises allowing the above enzyme of the present invention to react with L-glutamic acid in the presence of oxygen and water, and measuring the consumption of oxygen or the formation of hydrogen peroxide, of ammonia or of α-ketoglutaric acid accompanying the reaction.

In another embodiment of the aforesaid method the sample to be analyzed may contain a large amount of L-aspartic acid so as to have a substantial influence on the analysis of L-glutamic acid in known methods, the method being carried out at a pH of preferably 5 to 6. It is preferred to have the L-glutamic acid oxidase immobilized on a carrier.

The present invention also provides a reagent for analysis of L-glutamic acid comprising the aforesaid L-glutamic acid oxidase.

Further, the present invention provides a kit for analysis of L-glutamic acid comprising the aforesaid L-glutamic acid oxidase and a detecting reagent for the reaction by said enzyme. Most suitably such reagent is one for detection of hydrogenperoxide, ammonia of α-ketoglutaric acid.

The present invention also provides a biosensor comprising a receptor portion for a substance to be detected comprising an enzyme and a transducer portion which detects the chemical or physical change in a sample to be analyzed by the action of said enzyme and transduces it to an electric signal, wherein there is used as the enzyme the L-glutamic oxidase according to the invention.

The biosensor of the present invention, which employs an oxidase as the receptor portion of a biosensor can be manufactured very easily. Furthermore, the detecting means of the enzymatic reaction may be chosen from a variety of means known in the art depending on its purpose, such as a means for detecting reduction of oxygen which is a substrate or a means for detecting increase of hydrogen peroxide or ammonia which is the reaction product. Further, the biosensor of the present invention has by far higher specificity for L-glutamic acid as compared with the known biosensors and therefore can be used for analyzing L-glutamic acid selectively without interference by other amino acids even in a sample containing many kinds of amino acids.

By the use of the biosensor of the present invention as described above, not only can L-glutamic acid be analyzed in samples containing L-glutamic acid as its essential component such as foods, fermented liquors and others, but it is also possible to analyze enzyme activities of other substances through an enzyme reaction in which L-glutamic acid is liberated, for example, in the reactions systems of glutaminase, glutamic acid racemase, glutamic acid-oxaloacetic acid transaminase (GOT), glutamic acid-pyruvic acid transaminase (GPT), and γ-glutamyl transpeptidase (γ-GTP).

Brief description of the drawings

In the drawings:

Fig. 1 is a graph showing for comparison purposes the active pH ranges of the enzyme of the present invention (solid line) and of the known enzyme (broken line);

Fig. 2 is a graph showing stable pH ranges (37°C, maintained for 60 minutes) of the enzyme of the present invention (solid line) and the known enzyme (broken line);

Fig. 3 is a graph showing stable pH range (45°C, maintained for 15 minutes) of the enzyme of the present invention;

Fig. 4 is a graph showing stable pH range (60°C, maintained for 15 minutes) of the enzyme of the present invention;

Fig. 5 is a graph showing the optimum acting temperature range of the enzyme of the present invention;

Fig. 6 is a graph showing stable temperature ranges of the enzyme of the present invention for different pH values (solid line) and the known enzyme (broken line);

Fig. 7 is a graph showing the UV-absorption spectrum of the enzyme of the present invention;

Fig. 8 and Fig. 9 are graphs respectively showing the calibration curves of L-glutamic acid when a phenol solution and a dimethylaniline solution are used as the reagent B in the kit of Example B1;

Fig. 10 is a graph showing the calibration curve of L-glutamic acid in Example B2;

Fig. 11 is a graph showing the calibration curve of L-glutamic acid in Example B3;

Fig. 12 is a graph showing the calibration curve of GOT activity in Example B4;

Fig. 13 is a graph showing the calibration curve of GPT activity in Example B4;

Fig. 14 is a graph showing the calibration curve of L-glutamic acid in Example B5;

Fig. 15 is a graph showing the calibration curve of L-glutamic acid obtained by the use of the enzyme electrode of Example B6;

Fig. 16 is a graph showing the calibration curve of glutaminase activity in Example B7;

Fig. 17 is a schematic diagram showing the L-glutamic acid sensor according to the present invention;

Fig. 18 is a diagram showing schematically the L-glutamic acid analyzer employing the L-glutamic acid sensor shown in Fig. 17;

Fig. 19 is a graph showing the correlation between sodium L-glutamate concentration and the current reduction value when the device shown in Fig. 18 is used;

Fig. 20 is a graph showing responses at respective temperatures when the device shown in Fig. 18 is used;

Fig. 21 is a graph showing responses at respective pH values when the device shown in Fig. 18 is used;

Fig. 22 is a graph showing the correlation between sodium L-glutamate concentration and the current value when the L-glutamic acid analyzer described in Example C2 is used;

Fig. 23 is a graph showing the correlation between the amounts of sodium L-glutamate added to soy cause and recovered when the device shown in Fig. 18 is used; and

Fig. 24 is a graph showing the correlation between the amounts of sodium L-glutamate added to soy sauce and recovered when the L-glutamic acid analyzer described in Example C2 is used.

Detailed description of the invention

(I) Enzyme of the present invention:

The enzyme of the present invention may be any L-glutamic acid oxidase which has been derived from Streptomyces SP.X-119-6 (FERM P-6560) regardless of its preparation method.

As an example for the enzyme obtainable from the cultured product of this strain the properties and the method for preparation of this exemplary enzyme are detailed below.

(A) Enzymological and physicochemical properties of the enzyme of the present invention

The purified enzyme sample of the L-glutamic acid oxidase prepared according to the method of Example A1 hereinafter described has enzymological and physicochemical properties as set forth below.

(1) Action:

The enzyme of the present invention, when employing L-glutamic acid as substrate, demands 1 mol of oxygen and 1 mol of water per 1 mol of L-glutamic acid, and forms 1 mol of α-ketoglutaric acid, 1 mol of ammonia and 1 mol of hydrogen peroxide, as shown in the following reaction scheme.

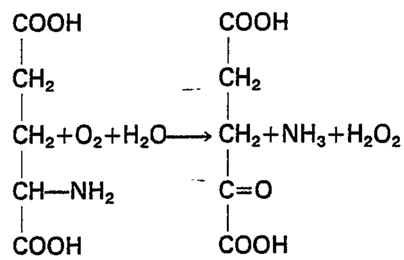

L-Glutamic acid α-Ketoglutaric acid

(2) Substrate specificity:

Table 1 shows the results obtained when the purified preparation of the enzyme of the present invention was caused to catalyze the oxidation of various amino acids. The concentration of each substrate was 10 mM, and the reactions were carried out at pH 7.4 (0.1 M potassium phosphate buffer) and pH 6.0 (0.1 M acetate buffer). The enzyme activities were measured according to the oxygen electrode method as hereinafter described and expressed as the relative values of activities to L-glutamic acid.

TABLE 1

| Substrate | Relative activity (%) | |
|---|---|---|
| | pH 7.4 | pH 6.0 |
| L-Glutamic acid | 100.0 | 100.0 |
| D-Glutamic acid | <0.1 | <0.1 |
| L-Aspartic acid | 0.6 | <0.1 |
| L-Glutamine | <0.1 | <0.1 |
| L-Asparagine | <0.1 | <0.1 |
| Glycine | <0.1 | <0.1 |
| L-Alanine | <0.1 | <0.1 |
| L-Valine | <0.1 | <0.1 |
| L-Leucine | <0.1 | <0.1 |
| L-Isoleucine | <0.1 | <0.1 |
| L-Serine | <0.1 | <0.1 |
| L-Threonine | <0.1 | <0.1 |
| L-Phenylalanine | <0.1 | <0.1 |
| L-Tyrosine | <0.1 | <0.1 |
| L-Proline | <0.1 | <0.1 |
| L-Lysine | <0.1 | <0.1 |
| L-Ornithine | <0.1 | <0.1 |
| L-Histidine | <0.1 | <0.1 |
| L-Arginine | <0.1 | <0.1 |
| L-Cysteine | <0.1 | <0.1 |
| L-Methionine | <0.1 | <0.1 |

As described above, the enzyme of the present invention has a high substrate specificity for L-glutamic acid. For other amino acids, it exhibits only a little activity (0.6%) for L-aspartic acid at pH 7.4, exhibiting substantially no activity for other L-amino acids including L-glutamine and L-histidine and for D-glutamic acid, also exhibiting substantially no activity even for L-aspartic acid at pH 6.0.

As contrasted to the enzyme of the present invention, the known enzyme as described above exhibits no activity for L-aspartic acid (0.1% or less), but exhibits activities of 8.4% for L-glutamine and 6.8% for L-histidine, respectively. Thus, both are different from each other in substrate specificity.

The enzyme of the present invention has a km value for L-glutamic acid of $2.1\times10^{-4}$ M at pH 7.4 and a km value for L-aspartic acid of $2.9\times10^{-2}$ M at pH 7.4.

(3) Assay of activity:

The activity of the enzyme of the present invention was assayed according to the oxygen electrode method. That is, 1 ml of 0.1 M potassium phosphate buffer (pH 7.4) containing 10 mM sodium L-glutamate was charged into an oxygen electrode cell and 10 µl of an enzyme solution was added thereto to measure the oxygen consumption rate. One unit of enzyme was determined as the amount of enzyme which

consumes 1 μ mol of oxygen per one minute at 30°C in the absence of catalase (unit: hereinafter abbreviated as "U").

Since the dissolved oxygen concentration is reduced with elevation of the temperature, the above method cannot be used for activity assay at higher reaction temperatures. In such a case, the activity assay is conducted according to the MBTH method [Anal. Biochem., *25*, 228 (1968)]. That is, a reaction mixture containing sodium L-glutamate, catalase and the enzyme of the present invention is incubated at an appropriate temperature for 20 minutes and the reaction is terminated with addition of trichloroacetic acid (TCA). To the terminated reaction mixture are added an acetate buffer (pH 5.0) and 3-methyl-2-benzothiazolinonehydrazone hydrochloride (MBTH) for incubation at 50°C for 30 minutes, followed by cooling to room temperature, and thereafter the absorbance at 316 nm is measured to determine quantitatively the α-ketoglutaric acid formed from the calibration curve.

(4) Optimum pH:

The optimum pH is around pH 7 to 8.5 as shown in Fig. 1. The enzyme activities at respective pH values were assayed at 30°C by using sodium L-glutamate as a substrate in 0.2 M acetate buffer (pH 3.5—6.0), 0.2 M potassium phosphate buffer (pH 6.0—8.5) and 0.2 M glycine-sodium chloride-sodium hydroxide buffer (pH 8.5—12.0).

In Fig. 1, for the purpose of comparison with respect to the optimum pH between the enzyme of the present invention and the known enzyme, both, of the pH activity curves of the enzyme of the present invention (solid line) and the known enzyme (broken line: reference is made to Fig. 1 in Japanese Patent Laid-Open Publication No. 43685/1982) are shown.

As is apparent from Fig. 1, the enzyme of the present invention is different from the known enzyme also in the optimum pH.

Also, when employing aspartic acid as the substrate, the acting pH range is narrow, the optimum pH being 7 to 8, and the enzyme has substantially no action on L-aspartic acid at pH of 6.0 or less or at pH 10.0 or more (at pH 6.0, 0.1% or less of the relative activity for glutamic acid).

(5) pH stability:

After maintaining the enzyme at respective pH values of from pH 3.5 to 11.5, under the conditions of 37°C for 60 minutes, 45°C for 15 mintues and 60°C for 15 minutes, the enzyme activity for glutamic acid was assayed at pH 7.4.

As a result, under the conditions maintained at 37°C for 60 minutes, the enzyme was stable at a pH range from 5.5 to 10.5 (Fig. 2, solid line); stable at a pH range from 5.5 to 9.5 under the conditions of 45°C for 15 minutes (Fig. 3); and stable at a pH range from 5.5 to 7.5 under the conditions of 60°C for 15 minutes (Fig. 4).

In Fig. 2, for the purpose of comparison relative to pH stability between the enzyme of the present invention and the known enzyme, both of the pH stability curves of the known enzyme (broken line: reference is made to Fig. 2 in Japanese Patent Laid-Open Publication No. 43685/1982) and the enzyme of the present invention are shown.

As is apparent from Figs. 2, 3, and 4, when stable pH ranges are compared between the enzyme of the present invention and the known enzyme, both are clearly different from each other, the former being stable at a wider pH range as compared with the latter.

(6) Suitable acting temperature range:

At respective temperatures of 30°C to 80°C, the reactions were carried out for 20 minutes with the use of sodium L-glutamate as a substrate, and the enzyme activity was assayed according to the MBTH method as described above.

As a result, the suitable acting temperature range of the enzyme of the present invention was found to be 30 to 60°C, with the optimum acting temperature being around 50°C (Fig. 5).

(7) Thermal stability:

After maintaining the enzyme at respective temperatures of 40°C to 90°C under the respective conditions of pH 5.5, pH 7.5 and pH 9.5, for 15 minutes, the enzyme activity for glutamic acid was assayed at pH 7.4.

As a result, the enzyme was found at pH 5.5 to be stable up to 65°C, exhibiting a residual activity of about 50% at 85°C (Fig. 6, ▲—▲). At pH 7.5, it was stable up to 50°C, exhibiting a residual activity of about 60% at 75°C (Fig. 6, ●—●). At pH 9.5, it was stable up to 45°C, exhibiting a residual activity of about 50% at 70°C (Fig. 6, ■—■).

For the purpose of comparison with regard to thermal stability between the enzyme of the present invention and the known enzyme, the temperature stability curve of the known enzyme (broken line: reference is made to Fig. 3 in Japanese Patent Laid-Open Publication No. 43685/1982) and that of the enzyme of the present invention are shown in the same drawing.

As is apparent from Fig. 6, the enzyme of the present invention has a higher thermal stability than the known enzyme.

(8) Inhibition, activation and stabilization:

For examination of the effects of various additives on the enzyme activity of the present invention, enzymatic reaction was carried out in a reaction mixture (pH 7.4) containing each of the substances shown in Table 2 at a concentration of 1 mM.

The results are as shown in Table 2.

TABLE 2

| Additives | Relative activity | Additives | Relative activity |
|---|---|---|---|
| (No addition) | 100 | $MnSO_4$ | 102.1 |
| KCl | 111.1 | $CoSO_4$ | 100.7 |
| NaCl | 95.8 | $Al_2(SO_4)_3$ | 93.8 |
| KI | 100.7 | EDTA[1] | 96.5 |
| NaF | 107.6 | NEM[2] | 94.4 |
| $CaCl_2$ | 100.0 | PCMB[3] | 55.6 |
| $CuCl_2$ | 100.7 | o-phenanthroline | 97.8 |
| $BaCl_2$ | 95.1 | α,α'-dipyridyl | 94.4 |
| $NiCl_2$ | 96.5 | $NaN_3$ | 100.6 |
| $StCl_2$ | 97.2 | DDTC[4] | 100.7 |
| $Li_2SO_4$ | 93.8 | Tiron[5] (trade mark) | 100.7 |
| $ZnSO_4$ | 90.3 | | |

1) EDTA: ethylenediaminetetraacetic acid
2) NEM: N-ethylmaleimide
3) PCMB: p-chloromercuribenzoate
4) DDTC: diethyldithiocarbamate
5) Tiron: 4,5-dihydroxy-1,3-benzenedisulfonic acid disodium salt

As is apparent from Table 2, the activity of the enzyme of the present invention is inhibited by about 45% by p-chloromercuribenzoate but is not inhibited at all by cupric chloride and diethyldithiocarbamate. On the other hand, the activity of the known enzyme is completely inhibited by cupric chloride and diethyldithiocarbamate. Therefore, both of the enzymes are different from each other also with respect to the effect by inhibitors.

At present, no activator and stabilizer have been found for the enzyme of the present invention.

(9) UV-absorption spectrum (see Fig. 7):

$\lambda_{max}$: 273 nm, 385 nm, 465 nm

Shoulder: around 290 nm, around 490 nm

(10) Coenzyme:

The absorption spectrum of the supernatant obtained by heat treatment or trichloroacetic acid (TCA) treatment of the enzyme of the present invention was identical with that of flavin adenine dinucleotide (FAD). The supernatant activated the apoenzyme of D-amino acid oxidase, and therefore the coenzyme of the enzyme of the present invention was found to be FAD.

The yellow compound in the supernatant was also identified as FAD from the Rf value in thin layer chromatography.

FAD was estimated to exist in an amount of 2 mol per 1 mol of the enzyme of the present invention.

(11) Polyacrylamide gel electrophoresis:

The purified enzyme of the present invention exhibited a single band.

(12) Molecular weight:
The enzyme of the present invention was estimated to have a molecular weight of 135,000±10,000 according to the gel filtration method by the use of Sephadex G-200 (produced by Pharmacia Fine Chemicals, Inc.).

(13) Isoelectric point:
The isoelectic point was measured by electrophoresis by the use of Ampholine (produced by LKB Co.) to find that pI was 6.2.

(14) Crystalline structure and elemental analysis:
The enzyme of the present invention was not crystallized, and no measurement has been performed.

(15) Purification method:
The enzyme of the present invention can be purified according to procedures involving salting out, isoelectric point precipitation, precipitation by an organic solvent, adsorption with diatomaceous earth, activated charcoal, etc., various chromatographies, and others. Examples of the purification methods are shown in Example A1.

(B) Preparation of the enzyme of the present invention
The method for producing the enzyme of the present invention will now be described in detail.

Microorganism employed
The microorganism employed in the production of the enzyme of the present invention belongs to the genus of *Streptomyces* and is the X-119-6 strain isolated as a single strain from the soil sample in Tōnoshō-machi, Katori-gun, Chiba-ken, Japan. The properties of this strain are described below.

A. Microscopic observation:
Aerial mycelia are straight with widths of 0.9 to 1.0 μ, exhibiting simple branching. Sporophores consist of a number of chains of spores, forming spirals of 2 to 5 rotations. Spores are somewhat ellipsoidal with sizes of 0.9—1.0×1.1×1.2 μ, and the surface is observed by electron microscope to have a spiny structure. No breaking of the basal mycelia is observed.

B. Observation by naked eye:
The results of observation by naked eye after growth on various media (30°C, 16 days' cultivation) are as follows.

(1) Sucrose-nitrate agar medium:
Its growth is poor. The basal mycelia are grayish brown and do not penetrate into the agar, and the aerial mycelia are powdery and spread radially on the agar. The aerial mycelia are grayish brown, with formation of gray spores. No formation of pigment into the medium is observed.

(2) Glucose-asparagine agar medium:
Its growth is good. The basal mycelia are white yellow, penetrate into the agar, and are also slightly raised. The aerial mycelia are white with no formation of pigment into the medium.

(3) Glycerin-asparagine agar medium:
Its growth is good. The basal mycelia are white yellow, penetrate into the agar, and are also raised. No aerial mycelium, is formed, and no formation of pigment into the medium is observed.

(4) Starch-inorganic salts agar medium:
Its growth is good. The basal mycelia are white yellow, penetrate into the agar, and are also raised. The aerial mycelia are white and abundant with formation of gray spores. No pigment formation into the medium is observed.

(5) Tyrosine-agar medium:
Its growth is good. The basal mycelia are white yellow. The aerial mycelia are white and abundant with formation of gray spores. No pigment formation into the medium is observed.

(6) Nutrient-agar medium:
Its growth is very good. The basal mycelia are white yellow, penetrate into the agar, and are also raised. The aerial mycelia are white, with no formation of spore being observed. No pigment formation into the medium is observed.

(7) Yeast-malt agar medium:
Its growth is very good. The basal mycelia are white yellow, penetrate into the agar, and are also

raised. The aerial mycelia are white and abundant with formation of gray spores. No pigment formation into the medium is observed.

(8) Oatmeal-agar medium:
Its growth is very good. The basal mycelia are white, penetrate into the agar, but are not raised on the medium. The aerial mycelia are white and abundant with formation of gray spores. No pigment formation into the medium is observed.

C. Physiological properties:
Growth temperature range is 8 to 40°C, the optimum temperature being around 35°C.
In both of the tyrosine-agar medium and the peptone-yeast-iron-agar medium, no melanin-like pigment is formed, gelatin is slightly liquefied, and starch is hydrolyzed.

D. Assimilabilty of various carbon sources:
Utilizations of various carbon sources on the Pridham-Gottrieb agar medium are as shown in Table 3.

TABLE 3

| Carbon source | Utilization* |
|---|---|
| D-Glucose | + |
| D-Xylose | − |
| L-Arabinose | + |
| L-Rhamnose | − |
| D-Fructose | + |
| Raffinose | + |
| Mannitol | + |
| Inositol | + |
| Sucrose | + |

*) +: utilized, −: not utilized.

The above properties may be summarized as follows. That is, aerial mycelia are spiral, the surfaces of the spores being spiny. Growth on media exhibits white yellow color or grayish brown color, aerial mycelia being colored white to grayish brown, and no formation of soluble pigment and melamin-like pigment is observed. Furthermore, starch hydrolyzability is rather strong.

On the basis of these results and assimilability of carbon sources shown in Table 3, the present microorganism strain was classified according to the taxonomic system in Bergey's Manual of Determinative Bacteriology, eighth edition (1974), whereby it was found that the present microorganism strain belongs to the genus *Streptomyces*, but no known species sufficiently coinciding in characteristics with the present strain was found, and hence the present strain was identified to be a new microorganism strain and named *Streptomyces* sp. X-119-6.

The present microorganism strain was deposited at the Fermentation Research Institute, Agency of Industrial Science and Technology (FRI), 1—3, Rigashi 1-chome, Yatabe-machi, Tsukuba-gun, Ibaraki-ken, Japan on June 5, 1982, and given the deposition number FERM P-6560. This strain was also delivered directly from FRI to American Type Culture Collection, 12301 Parklawn Drive, Rockville, Maryland, U.S.A. and was given there the deposition number ATCC 39343 on April 26, 1983.

The above microorganism strain is one example of the microorganism strains having high capability of producing the enzyme of the present invention, and the microorganism to be used in the present invention is not limited thereto. It is also possible to use suitably any of the mutant strains highly capable of producing the enzyme of the present invention obtained by subjecting the microorganism producing the enzyme of the present invention to conventional microorganism mutating methods such as physical treatment by UV-ray, X-ray or γ-ray irradiation, chemical treatments with reagents such as nitrosoguanidine, etc..

Cultural method and conditions

The cultural method and conditions for cultivating the above microorganism to be used in the present invention are not particularly limited, as long as the microorganism can sufficiently grow and the enzyme of the present invention can be sufficiently produced, but it is preferred to use a solid cultivation method or similar method.

The solid medium to be used in solid cultivation is not different in any way from those conventionally used. That is, the solid medium is mainly composed of one kind or more kinds of natural solid materials such as wheat bran, defatted soy bean, rice bran, corn, rapeseed dregs, wheat, rice, rice hulls, etc., further containing, if desired, nutrient sources assimilable by the microorganism employed in the present invention, as exemplified by carbon sources such as glucose, sucrose, arabinose, fructose, mannitol, inositol, soluble starch, ethanol, etc., nitrogen sources such as various amino acids, peptone, soybean powders, protein hydrolysates, corn steep liquor, meat extract, yeast extract, various ammonium salts, various nitrates, urea, etc., growth promoters exemplified by salts such as various sodium salts, potassium salts, calcium salts, manganese salts, magnesium salts, zinc salts, iron salts, phosphates, sulfates, etc., and vitamins such as thiamine, riboflavin, nicotinic acid, pantothenic acid, biotin, p-aminobenzoic acid, cyanocobalamin, etc. These media may also be granulated in suitable formulations, sizes and shapes. Such a solid medium may be sterilized or denatured according to conventional procedures and then inoculated with a seed microorganism to carry out solid cultivation.

It is also possible to employ a cultivation method other than the above method, as long as the microorganism employed can proliferate and produce well the enzyme of the present invention, such as the method in which a liquid medium is absorbed into or coated over a suitable carrier such as sponge, etc. (see Japanese Patent Laid-Open Publication No. 14679/1974), and a seed microorganism is inoculated into the medium to be cultured therein.

The cultural conditions are not particularly limited and may be selected optimally for production of the enzyme depending on the kind of the microorganism employed. Generally, cultivation may be conducted under the conditions of, for example, 20—30°C, pH 5—7 and 5—15 days.

Collection of the enzyme of the present invention

The enzyme of the present invention produced by cultivation of the microorganism employed may be separated by extraction from the cultured product, namely, the medium and/or the cultured microorganism cells, according to a suitable extraction method. The enzyme may be used as the crude enzyme solution or purified according to a conventional enzyme purification method to a purification degree which depends on the purpose of use.

The extraction method is not particularly limited but may be a conventional method. For example, extraction from the solid cultured product is ordinarily conducted with water or a buffer. The enzyme of the present invention in microorganism cells is extracted after crushing the microorganism cells in a conventional manner and solubilizing the enzyme.

(II) Analysis of L-glutamic acid:

1) Sample to be analyzed

The term "sample to be analyzed" used in the present description means (A) a sample which contains or is expected to contain L-glutamic acid as its component and in which the L-glutamic acid content or its presence or absence is to be analyzed, or (B) a sample containing a reaction system which liberates or is expected to liberate L-glutamic acid, an enzyme activity participating in the system or the content of a substance converted to L-glutamic acid or the presence or absence of these substances being to be analyzed by measurement of the change in the L-glutamic acid content in the system.

Examples of samples classified into the above (A) are foods (e.g., liquid seasoning foods such as soy sauce, amino acid seasoning, various extracts, liquid essence of soup stock, etc.; alcohol-containing foods such as *sake*, sweet *sake*, etc.; extracts from solid foods such as fish pastes, sausages, hams, etc.), and biological samples (urine, blood, etc.). On the other hand, examples of samples classified into (B) are systems containing an enzyme producding L-glutamic acid as the reaction product such as glutaminase, glutamic acid racemase, GOT, GPT, γ-GTP, etc. and substrates therefor, and systems containing the aforesaid system and one or more kinds of other enzyme systems which can be coupled with the aforesaid system.

2) Analysis of L-glutamic acid

The method for analysis of L-glutamic acid in the present invention comprises the enzymatic reaction system of L-glutamic acid in a sample with the enzyme of the present invention at the pH of from pH 5 to 9, particularly the enzymatic reaction system at pH 5 to 6 for L-glutamic acid in a sample in which a large amount of L-aspartic acid is present, and a detection system of an indicator substance, which is consumed or formed with progress of the reaction, for quantitative or qualitative analysis.

Enzymatic reaction

The conditions of enzymatic reaction for analysis of L-glutamic acid are as follows.

The reaction pH may be any pH at which the enzyme of the present invention is not inactivated and can

act sufficiently on L-glutamic acid. Further, if the detection system of indicator substance depends on pH, it is preferable to set appropriate pH. Ordinarily, the enzymatic reaction is carried out at a pH of from 5 to 9. In the case when L-aspartic acid is contained in a sample in such a large amount as to have an influence on the analysis of the L-glutamic acid, the enzyme of the present invention may act to a little extent on L-aspartic acid at the optimum pH. However, even in such a sample, L-glutamic acid can be specifically analyzed by carrying out the reaction under the conditions chosen so that the enzyme has no action on the L-aspartic acid. As such conditions, the reaction condition of from pH 5 to 6 is preferred.

For the purpose of maintaining the pH of the enzymatic reaction in a desirable range, it is preferable to use various buffers as the reaction medium. Any buffer which can maintain the aforesaid pH range, does not inhibit the activity of the enzyme of the present invention, and has no influence on the detection system of indicator substance can be used. For example, illustrative of such buffers are phosphate buffer, Tris-hydrochloride buffer, acetate buffer, citrate buffer, and Belonal buffer.

The reaction temperature is not particularly limited. Those skilled in the art can easily determine the reaction temperature by considering the optimum temperature and stable temperature for the enzyme of the present invention, and the detection system of indicator substance employed.

It is possible in carrying out the reaction to use the enzyme of the present invention as a soluble enzyme or an immobilized enzyme prepared according to the methods practiced in general such as the inclusion method (the lattice type, the microcapsule type, etc.), the carrier binding method (e.g., the covalent bonding method, the ion bonding method, or the physical adsorption method), and the cross-linking method.

The modes of immobilization are not limited (see "Immobilized Enzyme", edited by Ichiro Chihata, published on March 20, 1975, by Kodansha Co., Ltd. Examples of materials suitable for the carrier or the support are cellulose (e.g., Cellophane, filter papers), cellulose derivatives including acetyl cellulose derivatives, nitrocellulose (e.g., collodion), various ion-exchange cellulose derivatives (e.g., DEAE-cellulose, TEAE-cellulose, ECTEOLA-cellulose, CM-cellulose, P-cellulose), polysaccharides such as starch, dextran derivatives [e.g., Sephadex (trade name)], agarose, mannan (e.g., konjak mannan), chitosan, carageenan, alginic acid, xanthan gum, and agar, proteins such as collagen, gelatin, fibroin, keratin, albumin, and gultene, polymeric gels such as polyacrylamide, polyvinyl pyrrolidone, and polyvinyl alcohol, synthetic organic polymers such as polyvinyl chloride, polymethyl methacrylate, polyacrylonitrile, polystyrene, polyaminostyrene, polyethylene, polypropylene, polyurethane, polycarbonate, polyamide (e.g., nylon, polyamino acid), fluorine resins [Teflon (trade name)], silicone resins, photosensitive resins, adsorbent resins, and ion-exchange resins, inorganic materials such as glass, silica, ceramic, alumina, kaolinite, bentonite, calcium phosphate, hydroxyapatite, and activated charcoal. These materials may be used as they are or after introduction of functional groups which can react with the enzyme protein or activation of the functional groups.

According to the inclusion method, the enzyme can be captured within the lattice of the gel such as of polymeric gels, cellulose derivatives, polysaccharides or proteins. With the method, the enzyme may be coated with a semi-permeable skin membrane such as that of an organic polymeric material or cellulose derivative to make a microcapsule. In the case of the carrier binding method, immobilization can be accomplished by covalent bonding onto a suitable carrier by the diazo method, the peptide method, the alkylation method or the cross-linking method (with glutaraldehyde, hexamethylene diisocyanate, etc.), by ionic bonding onto a carrier having ion-exchange groups or by physical adsorption. Also, according to the cross-linking method, immobilization can be carried out by cross-linking between enzymes with the use of a reagent having two or more functional groups such as glutaraldehyde, isocyanate derivatives (hexamethylene diisocyanate, toluene diisocyanate, etc.), isothiocyanate derivatives (hexamethylene diisothiocyanate, etc.), N,N'-ethylenebismaleinimide, N,N'-(1,2-phenylene)bismaleinimide, N,N'-(1,4-phenylene)bismaleinimide, and N,N'-polymethylene-bisiodoacetamide. Immobilized enzymes can also be prepared by practicing two or more of these methods in combination.

According to the methods as described above, the enzyme of the present invention can be immobilized and prepared in any suitable form such as membrane, gel, granule, chip, powder, microcapsule, tube, fiber, hollow fiber, and vessel.

Detection system of indicator substance

The indicator substance for analysis of L-glutamic acid according to the method of the present invention includes oxygen consumed by the enzymatic reaction as well as hydrogen peroxide, ammonia, and α-ketoglutaric acid formed.

Detection of each indicator substance may be performed according to any desired method, and the present invention is not limited in its detection method. That is, a known method is usually adopted as the detection method, but various methods to be developed in the future may also be considered to be available.

The detection methods for respective indicator substances are as follows.

(1) Detection of oxygen:

As a method for detection of oxygen consumed, there have been known manometric methods ("Course of Biochemical Experiments 5, Enzymatic Research Method (A)", pp. 35—41, published on August

20, 1975, Tokyo Kagaku Dojin Co., Ltd.) and oxygen electrode method ("Measurement of Oxygen by the Electrode Method", edited by Bunji Hagiwara, published on November 20, 1977, by Kodansha Co., Ltd.). Either method can be employed in the present invention.

Oxygen electrodes have basic structures employing as the acting electrode a noble metal such as platinum, gold, or iridium and as the reference electrode an electrode of silver, silver/silver chloride system, saturated calomel, lead, zinc, aluminum, etc., with an electrolyte (an alkali solution such as of potassium hydroxide, sodium hydroxide, etc.) existing between these electrodes. More specifically, composite type electrodes such as Clark type electrodes (Fig. 17) or separation type electrodes may be used. Their system may be either the polarographic system or the Galvanic cell system.

In measuring oxygen by means of an oxygen electrode, the dissolved oxygen in the enzymatic reaction mixture may be measured according to a conventional method. The measurement may also be conducted by the use of an enzyme electrode.

(2) Detection of hydrogen peroxide:

For detection of hydrogen peroxide formed, any of the electrochemical analytical method, the spectrophotometric analytical method, the fluorometric analytical method, the chemiluminescent analytical method, and others known in the art can be used.

As the electrochemical analytical method, the method in which a hydrogen peroxide detecting type electrode with a structure similar to the above oxygen electrode is employed. The analysis is conducted according to the measurement of current. For example, a highly suitable electrode is the Clark type hydrogen peroxide electrode which comprises an acting electrode of a noble metal such as platinum, a reference silver electrode, an electrolyte, and a hydrogen peroxide permeable diaphragm. Also, similarly as in the case of the oxygen electrode, it may be used as the enzyme electrode. In addition, as the electrochemical analysis hydrogen peroxide can be determined with an ion electrode on the basis of reduction of iodine ion accompanied by the reaction among hydrogen peroxide, peroxidase and iodine ion. A catalyst degrading hydrogen peroxide such as molybdate can also be used as well as peroxidase. This method is preferably practiced with the use of an enzyme electrode.

As the spectrophotometric analytical method, there are (1) the peroxidase method in which a color former to be oxidized is caused to react with hydrogen peroxide in the presence of peroxidase or a substance exhibiting similar activity, and the absorbance of the color formed by the reaction is measured; (2) the catalase method in which an alcohol is caused to react with hydrogen peroxide in the presence of catalase, the aldehyde formed is led to a color forming system, and the absorbance of the color formed is measured, or an aldehyde dehydrogenase is caused to act on the aldehyde formed in the presence of NAD, and the amount of the reduced form of NAD (NADH) formed is measured; (3) the chemical method in which any of Ti(IV)/xylenol orange system, Ti(IV)/4 - (2 - pyridylazo)resorcinol system, V(V)/xylenol orange system, etc., is employed and (4) other methods.

In the peroxidase method of (1), the coloration development is an oxidation reaction of a color former alone or an oxidative condensation reaction of a color former with a coupler. In the former method, as the color former, o - dianisidine, o - tolidine, o - toluidine, o - aminophenol, 2,4 - dichloroindophenol, benzidine, 3,3',5,5' - tetraalkylbenzidine (e.g. 3,3',5,5' - tetramethylbenzidine, etc.), 4 - methoxy - 1 - naphthol, 2,2' - azino - di(3 - ethylbenzothiazoline - 6 - sulfonic acid), guaiacum resin (guaiacol), N - (4 - antipyryl) - aniline derivatives, (e.g., N - (4' - antipyryl) - 2 - carboxyl - 4 - hydroxyaniline, etc.), p - hydroxyphenyl acetic acid, N,N - dimethyl - p - phenylenediamine, etc. can be used.

On the other hand, in the latter method, as the color former, 4 - aminoantipyrine and 4 - aminoantipyrine derivatives such as 4 - aminoantipyrineamide; phenylenediamine derivatives such as 4 - aminophenazone, 4 - amino - N,N - dimethylaniline, p - phenylenediamine and 4 - amino - N,N - diethyl - m - toluidine; 3 - methyl - 2 - benzothiazolinone hydrazone (MBTH) can be used. As the coupler, phenol type, aniline type or toluidine type compounds such as phenol, catechol, resorcin, hydroquinone, cresol, guaiacol, pyrogallol, orcinol, p - chrorophenol, p - bromophenol, 2,4 - dichlorophenol, 2,4 - dibromophenol, 2,4,6 - tribromophenol, aniline, N,N - dimethylaniline, N,N - diethylaniline, N - ethyl - (N - 3 - methylphenyl) - N - acetylenediamine, 3 - acetamino - N,N - diethylaniline, N,N - diethyl - m - toluidine, N - ethyl - N - (2 - hydroxy - 3 - sulfopropyl) - m - toluidine, N - glycyl - N - ethyl - m - toluidine, p - dimethylaminophenol, o - aminophenol, m - aminophenol, p - methylaminophenol, 2 - chloro - 6 - methylphenol, 4 - chloro - 3 - methylphenol, 3,5 - dichloro - 2 - hydroxybenzene sulfonic acid, naphthol type compounds such as 4 - halogeno - 1 - naphthol - 2 - sulfonic acid (e.g., 4 - chloro - 1 - naphthol - 2 - sulfonic acid, etc.), 1 - naphthol - 2 - sulfonic acid, 2 - amino - 5 - naphthol - 7 - sulfonic acid, 2,4 - dichloro - 1 - naphthol, 1 - hydroxy - 2 - naphthoic acid, 4,5 - dihydroxynaphthalene - 2,7 - disulfonic acid and the like; naphthylamine or derivatives thereof; quinoline type compounds such as hydroxyquinoline, aminoquinoline and the like can be used.

Examples of preferable combinations of a color former and a coupler are a combination of 4 - aminoantipyrine with phenol, N,N - dimethylaniline, N,N - diethylaniline or N - diethyl - m - toluidine, and a combination of 3 - methyl - 2 - benzothiazolinonehydrazone with N,N - dimethylaniline. The present invention is not limited to these kinds of color formers or combinations of a color former and a coupler, and any compound can be used as long as it can be quantitatively oxidized to develop color. As the peroxidase, an enzyme obtained from horseradish or sweet potato is ordinarily used, but any enzyme

exhibiting peroxidase-like activity can be employed without particular limitation. Further, a catalyst exhibiting activity similar to peroxidase may also be used.

In the catalase methods of (2) with color formation, methanol is ordinarily used as alcohol. The color formation is ascribable to the reaction of formaldehyde formed with a hydrazone [e.g. 3 - methyl - 2 - benzothiazolinonehydrazone, 4 - amino - 3 - hydrazino - 5 - mercapto - 1,2,4 - triazole (AHMT), etc.] in the presence of an oxidizing agent (e.g., sodium periodate, potassium cyanophenolate, ferric chloride, etc.) or the reaction of formaldehyde with acetylacetone and an ammonium salt.

There are also the method in which glutathione is used and led to the oxidized form of glutathione by hydrogen peroxide and glutathione peroxidase, which is in turn reduced by glutathione reductase in the presence of NADPH, and the quantity of NADPH oxidized is measured (Anal. Biochem. *76*, 184—191, 1976), and the method in which indigo carmine is discolored by oxidation by a copper ion-histamine system and the analysis is made from reduction in its color degree.

In one example of the fluorometric methods, homovanilic acid is caused to react with hydrogen peroxide in the presence of peroxidase to form 2,2′ - dihydroxy - 3,3′ - dimethoxybiphenyl - 5,5′ - diacetic acid, the fluorescent intensity of which is measured. In an analogous method, in place of homovanilic acid, p-hydroxyphenyl acetic acid, diacetylfluorescein derivatives (e.g. diacetylfluorescein, diacetyldichlorofluorecein, etc.) may be also used as the fluorescent reagent. In another method a fluorescent substance such as scopoletin, 3,5 - diacetyl - 1,2 - dihydrolutidine, etc. is oxidized by hydrogen peroxide/peroxidase to form a non-fluorescent substance, and the degree of the reduction in the fluorescence intensity is measured.

In the chemiluminescent analytical method, luminol is caused to react with hydrogen peroxide in the presence of peroxidase, and the amount of luminescence is measured (see Japanese Patent Laid-Open Publications Nos. 71399/1982 and 71400/1982). In an analogous method, isoluminol, pyrogallol, bis(2,4,6 - trichlorophenyl)oxalate, etc., may also be used in place of luminol, and hemoglobin, hematine, hemine, potassium ferricyanide (III), cobalt chloride may also be employed as the catalyst in place of peroxidase.

(3) Detection of ammonia:

Ammonia can be detected according to the micro-Kjeldahl method, the Nessler's reagent method, the indophenol method, the ninhydrine method, or the phenosafranin method. Electrochemical analysis may also be possible according to the method in which ammonium ion is analyzed by a cation selective electrode or the method in which ammonia is analyzed as ammonia gas by an ammonia gas electrode comprising a glass electrode having thereon a hydrophobic gas permeable film. Further, L-glutamic acid can be analyzed by detection of ammonia by an enzyme electrode comprising a combination of these electrodes with the enzyme of the present invention.

(4) Detection of α-ketoglutaric acid:

For detection of α-ketoglutaric acid, the method in which it is caused to react with 3 - methyl - 2 - benzothiazolinonehydrazone (MBTH), and absorbance of the product formed is measured, the method in which it is caused to react the 2,4 - dinitrophenylhydrazine, and absorbance of the product formed is measured, the method in which it is caused to react with o-phenylenediamine, and absorbance of the product formed is measured, and other known methods can be used.

3) Reagent for analysis

The reagent for analysis of the present invention is a reagent containing at least the enzyme of the present invention. That is, its form is not limited and it may be a soluble enzyme in a form of solution, powder or granule. Further, it may be an immobilized enzyme prepared by various methods on a carrier in the form of membrane, gel, granule, powder, chip, microcapsule, tube, fiber, hollow fiber, or vessel as mentioned previously. It may also incorporate, in addition to the enzyme of the present invention, buffers such as a liquid or powdery phosphate buffer, Tris-hydrochloride buffer, acetate buffer, citrate buffer, Belonal buffer, etc.; salts (e.g., sodium chloride); sugars (e.g., sucrose); polyhydric alcohols (e.g., glycerol, propylene glycol, sorbitol); and other suitable stabilizers, surfactants, etc.

In the analysis of L-glutamic acid, the above reagent for analysis may be used so as to obtain the necessary enzymatic activity depending on the various detection methods as described above. It is also possible to previously seal an aliquot of the reagents corresponding in amount to the respective detection methods in a vessel such as a reagent vial or ampoule.

4) Kit for analysis

The kit for analysis consists essentially of the above reagents for analysis and reagents for the detection of the enzymatic reaction. The reagents for detection are those necessary for measurement of the indicator substance as described above. That is, when hydrogen peroxide is used as the indicator substance, illustrative of the reagent for detection are, for example, a combination of peroxidase or a peroxidase-like active substance with a color former or a color former and a coupler, a combination of catalase or a catalase-like active substance with an alcohol and a reagent necessary for a color forming system or a reagent necessary for a coupling enzyme system, a combination of peroxidase or a peroxidase-like active substance with a fluorescence-emitting agent, and a combination of peroxidase or a

peroxidase-like substance with a luminous reagent. Examples of these reagents will be apparent from the description in the foregoing "Detection of hydrogen peroxide".

Similarly, when ammonia or α-ketoglutaric acid is used as an indicator substance, a reagent necessary for detection can be combined with the reagent for analysis containing the enzyme of the present invention to constitute a kit for analysis.

The reagent for analysis containing the enzyme of the present invention and the aforesaid reagent for detection may be all mixed to form a single reagent, or when there exist some components interfering with each other, the respective components may be divided separately into suitable combinations. These may be prepared either as solutions or powdery reagents, or they may be incorporated in an appropriate support such as filter papers or films to prepare test papers or films for analysis.

To the kit for analysis of the present invention may also be added a standard reagent containing a certain quantity of L-glutamic acid in addition to the reagents as described above.

As a preferable example of the kit for analysis of the present invention, a kit for analysis of L-glutamic acid by spectrophotometric detection of hydrogen peroxide may be mentioned. For example, in the case of a kit according to the peroxidase method, ordinarily 0.02 U or more/test of the enzyme of the present invention, 1 to 10 U/test of peroxidase and a suitable amount of the color former may be employed. When, as the color former, 4-aminoantipyrine and phenol or N,N-dimethylaniline are to be employed, these reagents are used in an amount of 1 mole or more, preferably 2 moles or more, per mole of hydrogen peroxide formed.

(III) Biosensor:

1) Constitution of sensor

In the present invention, the "biosensor" means a device for analyzing the amount of a substance to be detected in a sample, which comprises an enzyme as the receptor portion for distinguishing a substance to be detected and a transducer portion which is a signal transducing site for detecting the chemical or physical change accompanied with enzymatic reaction. [See "Kagaku no Ryoiki (Region of Chemistry), special No. 134, Biomaterial Science Vol. 1", pp. 69—79, April 20, 1982, published by Nankodo Co., Ltd.; and "Kagaku Kogyo (Chemical Industry)", Vol. 6, 1982, pp. 491—496]. As mentioned above, the biosensor employing an enzyme as the receptor portion is also called an "enzyme sensor", and particularly the enzyme sensor employing an electrochemical device as the transducer portion is also called an "enzyme electrode" (see "Ion Electrode and Enzyme Electrode", edited by Shuichi Suzuki, pp. 65—106, November 1, 1981, published by Kodansha; and "Region of Chemistry", Vol. 36, No. 5, pp 343—349, 1982). There are two types of enzyme sensor, one is the mounting type enzyme electrode in which a receptor portion is provided in contact with or adjacent to a transducer portion, and another is the reactor type enzyme sensor in which a receptor portion and a transducer portion are separated from each other.

The present invention constitutes a novel biosensor by using a specific L-glutamic acid oxidase which is a novel enzyme as the receptor portion as mentioned above, whereby it has attained an effectiveness superior by far to that of the prior art technique. Accordingly, the known techniques can be used in the present invention except for the use of the enzyme of the present invention, and, further, the techniques to be developed in the future can also be employed as long as they are suited for the purpose of the present invention.

2) Receptor portion

In order to use the above enzyme of the present invention as the receptor portion of the biosensor, the receptor portion must be constituted to make good contact and reaction of the enzyme with L-glutamic acid in a sample. To prevent a leak of the enzyme from the receptor portion, the enzyme of the present invention is ordinarily immobilized or adsorbed on a semipermeable membrane (e.g., a dialyzing membrane or ultrafiltration membrane) before it is provided for use.

Immobilization may be performed according to any method suitable for the purpose of use of the receptor portion in the biosensor, which is not particularly limited as long as it does not substantially inactivate the enzyme of the present invention and interfere with the enzymatic reaction [see "Immobilized Enzyme", edited by Ichiro Chihata, March 20, 1975, published by Kodansha Co., Ltd. and "Ion Electrode and Enzyme Electrode" (ibid.), pp. 65—77].

That is, in the preparation of immobilized enzyme used as the receptor portion, the immobilization method may be chosen suitably from the known methods such as the inclusion method (the lattice type, the microcapsule type, etc.), the carrier binding method (e.g., the covalent bonding method, the ion bonding method, or the physical adsorption method) and the cross-linking method. Examples of materials suitable for the carrier or the support as well as the details of each of the immobilization methods are as previously mentioned.

The immobilized enzyme obtained may be used as the receptor portion of the biosensor in the following modes. That is, in the case of the mounting type enzyme electrode, the immobilized enzyme preparation ordinarily in a form of membrane, gel, powder or microcapsule is placed on a permeable membrane which is in contact with the surface of an electrode. And then, the enzyme and electrode are covered with a substrate-permeable porous membrane (e.g., dialyzing membrane, ultrafiltration membrane). The three constituents described above: a permeable membrane, an immobilized enzyme and

a covered membrane, can be constructed into a laminated membrane or a heterogeneous membrane. On the other hand, in the case of a reactor type enzyme sensor, an immobilized enzyme generally in the form of a granule or powder is packed in a column or a tube. An immobilized enzyme prepared in a form of tube, membrane, hollow fiber or fiber can also be constituted as the receptor portion to make a reactor.

3) Transducer portion

In the aforesaid receptor portion, the substrate in a sample for analysis is oxidized by the enzyme of the present invention and at the transducer portion the chemical or physical change by the reaction is detected and transduced into an electrical signal. In the following description, reference is made to examples in which chemical changes are detected, but a physical change such as heat balance may also be detected by means of a thermistor or the like.

The chemical changes accompanying the enzymatic reaction are consumption of oxygen and L-glutamic acid and formation of hydrogen peroxide, ammonia and α-ketoglutaric acid in the reaction mixture. The change to be detected by the transducer portion is ordinarily consumption of oxygen, formation of hydrogen peroxide or formation of ammonia.

As the transducer for detecting consumption of oxygen and transducing it into an electrical signal, an oxygen electrode may be employed.

As the transducer for detecting hydrogen peroxide and transducing the result into an electrical signal, a hydrogen peroxide detecting type electrode is employed. More specifically, it is possible to use an electrode having a structure similar to that of the oxygen electrode as described above.

Also, by using as the receptor portion a catalyst degrading hydrogen peroxide such as a peroxidase or a molybdate together with the enzyme of the present invention and permitting hydrogen peroxide to react with iodine ions, the activity reduction of iodine ions can be detected to determine hydrogen peroxide. In this case, an iodine ion electrode can be used.

As the transducer for detecting ammonia and transducing the result into an electrical signal, a cation selective electrode can be employed when ammonia is to be analyzed as ammonium ion, while an ammonia gas electrode can be used when ammonia is to be analyzed as ammonia gas.

The mounting type enzyme electrode according to the present invention has a structure in which the aforesaid enzyme is mounted on a membrane of various electrodes described above, or integrally combined with such a membrane.

Also, the reactor type enzyme electrode according to the present invention comprises a reactor having the immobilized enzyme as the receptor portion and an electrode of those as described above which is away from the place for the enzymatic reaction.

The present invention is not limited with respect to the transducer detecting substances and the transducer systems.

Example A1

Into an Erlenmeyer flask of 500-ml capacity were charged 20 g of wheat bran and 16 ml of water, and the sterilization was conducted at 120°C for 30 minutes. Into the wheat bran medium thus prepared was inoculated *Streptomyces* sp. X-119-6 (FERM P-6560, ATCC 39343) and cultured at 28°C for 7 days to prepare seed culture.

Into each of 25 Erlenmeyer flasks of 5-liter capacity were charged 200 g of wheat bran and 160 ml of water, and after the sterilization at 120°C for 30 minutes, the above seed culture was inoculated and was cultured at 28°C for 2 days and further at 20°C for additional 2 weeks.

The cultured product obtained was immersed in 37.5 liters of water for one hour, filtered and further passed through diatomaceous earth to obtain about 34 liters of a crude enzyme solution. Ammonium sulfate was added to the crude enzyme solution to 50% saturation, and the precipitates formed were collected by centrifugation and dissolved in 3.9 liters of 0.02 M acetate buffer (pH 5.5). The resultant solution was heated at 57°C for 30 minutes. The heat treated enzyme solution was cooled to 5°C or lower, and then to this solution was added a two-fold amount of previously cooled ethanol. The precipitates thus formed were collected by centrifugation, dissolved in 0.02 M phosphate buffer (pH 7.4), and dialyzed against the same buffer overnight.

The precipitates formed during dialysis were removed by centrifugation. The supernatant was passed through a DEAE (diethylaminoethyl)-cellulose column (3.5×50 cm) equilibrated with the same buffer, and the enzyme adsorbed was eluted with the same buffer containing 0.35 M sodium chloride. The active fractions eluted were collected, and dialyzed against 0.05 M acetate buffer (pH 5.5) containing 0.05 M sodium chloride. The inner dialyzed solution was passed through a column (2×10 cm) of DEAE-Sepharose CL-6B (produced by Pharmacia Fine Chemicals, Inc.) enquilibrated with the same buffer, and the enzyme adsorbed was eluted with 0.05—0.75 M linear gradient of sodium chloride.

The active fractions eluted were collected, concentrated by dialysis, and then subjected to gel filtration by use of a Sephadex G-200 (produced by Pharmacia Fine Chemicals, Inc.) column (2.5×120 cm). The active fractions were collected and, after concentration, dialyzed against 0.02 M potassium phosphate buffer (pH 7.4). The inner dialyzed solution was centrifuged, and the supernatant was subjected to microfiltration, which was followed by lyophilization to obtain 30 mg of a purified preparation of L-glutamic acid oxidase (specific activity 55.1 U/mg-protein, Yield 18.4%).

Example B1

(1) Preparation of kit:

Reagent A: In 2 ml of 0.2 M potassium phosphate buffer (pH 6.5) were dissolved 1 mg of L-glutamic acid oxidase (10 U/mg), 5 mg of peroxidase (100 U/mg, obtained from horseradish, produced by Toyo Boseki Co., Ltd.) and 40 mg of 4-aminoantipyrine so as to be contained in one reagent vial. Lyophilization was then carried out according to a conventional method.

Reagent B: ① A solution of 40 mg of phenol in 100 ml of 0.1 M phosphate buffer (pH 6.5) was placed in a reagent vial or ② a solution of 100 mg of dimethylaniline in 100 ml of 0.1 M potassium phosphate buffer (pH 6.5) was placed in a reagent vial.

(2) Operational procedure:

In each test tube was apportioned 0.1 ml of the standard solution of sodium L-glutamate or water, and 0.9 ml of the solution prepared by dissolving the lyophilized product of the above reagent A into 100 ml of either solution ① or ② of the reagent B was added to each of the above test tube. Then the incubation was carried out aerobically at 37°C with shaking for 20 minutes. With the blind test with water as reference, the absorbance was measured at 500 nm when using the above solution ①, and at 565 nm when using the above solution ②. These calibration curves are shown in Fig. 8 (when Reagent B is ①) and Fig. 9 (when Reagent B is ②).

Example B2

(1) Preparation of reagents:

Color forming reagent: In 50 ml of 0.2 M potassium phosphate buffer (pH 7.4) were dissolved 20 mg of phenol, 20 mg of 4-aminoantipyrine and 3 mg of horseradish peroxidase (100 U/mg).

L-glutamic acid oxidase: In 30 ml of 0.02 M potassium phosphate (pH 7.4) was dissolved 300 µg (55 U/mg) of the purified enzyme (0.55 U/ml).

(2) Operational procedure:

In each test tube was apportioned 0.8 ml of the color forming reagent, and 0.1 ml of L-glutamic acid oxidase solution was added thereto, which step was followed by preincubation at 37°C for 5 minutes. After addition of 0.1 ml of the standard sodium L-glutamate solution (0—2) µmol/ml) or sample solution (soy sauce diluted 150-fold with water), the mixture was stirred well and incubated aerobically with shaking at 37°C for 20 minutes. With the blind test as reference, the absorbance for the standard sodium L-glutamate at 500 nm was measured to prepare the calibration curve shown in Fig. 10. The amount of L-glutamic acid in the above samples was determined from this calibration curve as compared with the values of the amount of L-glutamic acid in the same samples according to the prior art method using an L-glutamic acid decarboxylase (the method in which color reduction degrees of phenolphthalein are measured by Technicon Autoanalyzer). The results are shown in Table 4 (shown after Example B3). The correlative coefficient was $\gamma=0.997$, the regression equation being $y=1.005x-0.575$.

Example B3

In each test tube were apportioned 0.7 ml of 0.1 M potassium phosphate buffer (pH 7.4), 0.1 ml of a solution of catalase (1000 U/ml, obtained from bovine liver, produced by Sigma Chemical Co.) and 0.1 ml of a solution of L-glutamic acid oxidase (0.6 U/ml), which step was followed by preincubation at 37°C for 5 minutes. The reaction was initiated by adding 0.1 ml of the standard sodium L-glutamate solutions (0—5 mM) or 0.1 ml of sample solutions (the same diluted solutions of soy sauce products as in Example B2). After incubation aerobically with shaking at 37°C for 20 minutes, the reaction was terminated by additon of 0.1 ml of 25% trichloroacetic acid. To the reaction terminated mixture were added 1.9 ml of 1 M acetate buffer (pH 5.0) and 0.8 ml of 0.1% 3-methyl-2-benzothiazolinonehydrazone hydrochloride solution. After stirring the mixture, the incubation was carried out at 50°C for 30 minutes. After cooling to room temperature, with the blind test as reference, the absorbance at 316 nm was measured to prepare the calibration curve shown in Fig. 11. The amount of L-glutamic acid in the samples was determined from this calibration curve as compared with the values of the amount of L-glutamic acid in the same samples according to the prior art method using an L-glutamic acid decarboxylase. The results are shown in Table 4. The correlative coefficient was $\gamma=0.996$, the regression equation being $y=1.026\times-3.122$.

TABLE 4

| Samples No. | Example B2 mM | Example B3 mM | Prior art method mM |
|---|---|---|---|
| 1 | 90.0 | 94.0 | 90.7 |
| 2 | 108.5 | 105.0 | 105.7 |
| 3 | 96.0 | 96.0 | 97.2 |
| 4 | 39.0 | 39.0 | 40.7 |
| 5 | 81.0 | 81.0 | 80.0 |
| 6 | 72.0 | 69.0 | 72.2 |
| 7 | 76.5 | 72.0 | 74.3 |
| 8 | 99.0 | 94.5 | 98.6 |
| 9 | 115.5 | 117.0 | 116.4 |
| 10 | 97.5 | 100.0 | 100.0 |

Example B4

(1) Preparation of reagents:

Color forming reagent: In 50 ml of 0.2 M potassium phosphate buffer (pH 6.5) were dissolved 30 mg of phenol, 30 mg of 4-aminoantipyrine, 4 mg of horseradish peroxidase (100 U/mg) and 1 mg of L-glutamic acid oxidase (10 U/mg).

Substrate solution A: In 10 ml of 0.1 M potassium phosphate buffer (pH 7.0) were dissolved 200 mg of sodium L-aspartate and 15 mg of α-ketoglutaric acid.

Substrate solution B: In 10 ml of 0.1 M potassium phosphate buffer (pH 7.0) were dissolved 140 mg of L-alanine and 15 mg of α-ketoglutaric acid.

(2) Operational procedure:

① Assay of glutamic acid-oxaloacetic acid transaminase (GOT) activity:

In each test tube was apportioned 0.2 ml of substrate solution A, 0.1 ml of the standard enzyme solution (Behlinger Yamanouchi Co., Ltd., 380 U/mg) was added thereto; and the incubation was carried out at 37°C for 30 minutes, which was followed by termination of the reaction by addition of 0.1 ml of 25% trichloroacetic acid. To the reaction terminated mixture were added 0.1 ml of 1 M potassium phosphate buffer (pH 6.5) and 0.5 ml of the color forming reagent containing L-glutamic acid oxidase, and the incubation was carried out at 37°C for 20 minutes. With the blind test as reference, the absorbance at 500 nm was measured to prepare the calibration curve shown in Fig. 12.

② Assay of glutamic acid-pyruvic acid transaminase (GPT) activity:

In each test tube was apportioned 0.2 ml of substrate solution B, 0.1 ml of the standard enzyme solution (Behlinger Yamanouchi Co., Ltd., 140 U/mg) was added thereto; and the incubation was carried out at 37°C for 30 minutes, which was followed by termination of the reaction by addition of 0.1 ml of 25% trichloroacetic acid. The content of L-glutamic acid in the reaction terminated mixture was measured similarly as in ① to prepare the calibration curve shown in Fig. 13.

Example B5

One ml of 0.1 M acetate buffer (pH 5.5) containing L-glutamic acid oxidase (0.5 U/ml) was sealed in the cuvette of a Clark type oxygen electrode in which thermostat water at 30°C was circulated, and then 50 µl of L-glutamic acid standard solutions (0—2 mM) was injected thereinto for measurement of the oxygen consumed. With the results the calibration curve was prepared as shown in Fig. 14. The amount of L-glutamic acid in the samples (various products of soy sauce) was determined from this calibration curve as compared with the amount of the same samples obtained by the prior art method using an L-glutamic acid decarboxylase. The results are shown in Table 5. The correlative coefficient was γ=0.953, the regression equation being y=0.930+6.11.

TABLE 5

| Samples No. | Example B5 mM | Prior art method mM |
|---|---|---|
| 1 | 74.9 | 77.8 |
| 2 | 81.6 | 80.0 |
| 3 | 79.7 | 82.1 |
| 4 | 78.3 | 78.5 |
| 5 | 81.1 | 83.5 |
| 6 | 69.7 | 73.5 |
| 7 | 85.9 | 85.7 |
| 8 | 91.6 | 91.4 |
| 9 | 82.1 | 85.7 |
| 10 | 71.6 | 71.4 |
| 11 | 77.2 | 77.1 |
| 12 | 74.8 | 72.1 |
| 13 | 74.8 | 75.0 |
| 14 | 73.8 | 70.0 |
| 15 | 57.9 | 61.4 |

Example B6

L-Glutamic acid oxidase solution (0.5 ml) (12.5 U/ml) was added dropwise under suction onto a porous nitrocellulose membrane (produced by Tōyō Kagaku Sangyō Co., Ltd.; TM-5; pore size 0.1 μm, diameter 25 mm, membrane thickness 140 μm) to be immobilized thereon by adsorption. Then an L-glutamic acid oxidase membrane was obtained after drying.

The above immobilized enzyme membrane was cut into a disc (diameter 5.0 mm), which was then mounted on the gas-permeable membrane (Teflon membrane, membrane thickness 10 μm) of a diaphragm oxygen electrode (produced by Ishikawa Seisakusho Co., Ltd., U-2 Model), and further a cellulose dialyzing membrane was covered on the immobilized enzyme membrane and the electrode to prepare an L-glutamic acid sensor.

By the use of this sensor, current reduction value for sodium L-glutamate standard solution of known concentration (0.05—1.0 mM) was measured to prepare the calibration curve shown in Fig. 15.

Example B7

One ml of a 0.1 M acetate buffer (pH 5.5) containing 2 U of L-glutamic acid oxidase and 20 μmol of L-glutamine was sealed in the cuvette of a Clark type oxygen electrode in which thermostat water at 30°C was circulated, and 20 μl of glutaminase standard solutions (4—20 mU, obtained from *E. coli*) was injected thereinto for measurement of the oxygen consumption rate to prepare the calibration curve shown in Fig. 16.

Similarly, 50 μl of a suspension of soy sauce *koji* (obtained by homogenizing 10 g of soy sauce *koji* in 50 ml of 0.1 M phosphate buffer (pH 7.0) at 1,000 r.p.m. for five minutes and then adding the same buffer to fill up 300 ml) was injected thereinto for measurement of the oxygen consumption rate. The glutaminase activity per gram of soy sauce *koji* was found to be 3.7 U.

Example C1

L-Glutamic acid oxidase solution (0.1 ml) (448 U/ml) was added dropwise and solidified on a Cellophane membrane (membrane thickness 30 μm, 30 mm×30 mm square), and the resultant product was impregnated with 0.1 ml of 2.5% glutaraldehyde solution and then dried at 4°C overnight. The

membrane was washed with water until no substance absorbing at 280 nm was eluted to obtain an immobilized Cellophane membrane of L-glutamic acid oxidase.

The above immobilized enzyme membrane was cut into a piece of circular shape, which was mounted on an oxygen permeable membrane (Teflon membrane, membrane thickness 10 μm) of a diaphragm oxygen electrode (produced by Ishikawa Seisakusho Co., Ltd.), and further a cellulose dialyzing membrane (membrane thickness 50 μm, produced by Visking Co.) was covered on the immobilized enzyme membrane and the electrode to prepare an L-glutamic acid sensor (Fig. 17).

In Fig. 17, the diaphragm oxygen electrode comprises a cathode of platinum electrode 1, an anode of lead electrode 2, an internal liquid (electrolyte) 3 of a potassium hydroxide solution, and an oxygen permeable Teflon membrane 4 mounted on the platinum cathode surface. On the Teflon membrane is mounted the above immobilized enzyme membrane 5, which is further covered with a cellulose dialysis membrane 6 and fixed with an O-ring 7.

By the use of the above enzyme electrode, an L-glutamic acid analyzing device as shown in Fig. 18 was prepared. In Fig. 18, the sample solution 2 injected through inlet 1 is transferred by a peristaltic pump 4 together with oxygen saturated buffer 3 into a cell 5. The current reduction value measured by the L-glutamic acid sensor 6 is recorded on the recorder 7.

Sodium L-glutamate standard solution was injected into the above device for measurement of the current reduction value. The correlation between the sodium L-glutamate concentration and the current reduction was obtained as shown in Fig. 19.

With the use of the device, responses at respective temperatures were measured, and the result was obtained as shown in Fig. 20. Measurements were carried out by using 0.8 mM sodium L-glutamate solutions respectively at temperature of 20°C, 30°C and 40°C.

Similarly, responses to sodium L-glutamate solution (0.8 mM) at 30°C and different pH values were determined. And the results were obtained as shown in Fig. 21.

Furthermore, responses to various amino acids were measured, and the results were obtained as shown in Table 6. In the measurement, the concentration of the solutions of amino acids other than L-glutamic acid was 2.0 mM while that of L-glutamic acid solution was 0.4 mM. The results obtained by the measurement were shown as relative responses. The reaction was carried out in 0.1 M of acetic acid-sodium acetate buffer at pH 5.5 and 30°C.

TABLE 6

| Substrate | Relative response (%) |
|---|---|
| L-Glutamic acid | 100.0 |
| D-Glutamic acid | <0.1 |
| L-Aspartic acid | <1.0 |
| L-Glutamine | <2.0 |
| L-Asparagine | <1.0 |
| Glycine | <0.1 |
| L-Alanine | <0.1 |
| L-Valine | <0.1 |
| L-Leucine | <0.1 |
| L-Isoleucine | <0.1 |
| L-Serine | <0.1 |
| L-Threonine | <0.1 |
| L-Phenylalanine | <0.1 |
| L-Tryptophan | <0.1 |
| L-Proline | <0.1 |
| L-Lysine | <0.1 |
| L-Ornithine | <0.1 |
| L-Histidine | <0.1 |
| L-Arginine | <0.1 |
| L-Cysteine | <0.1 |
| L-Methionine | <0.1 |

When the above sensor was preserved in 0.02 M phosphate buffer at 3°C, and responses to sodium L-glutamate solutions were measured similarly as described above on the respective elapsed days, it was found that the initial response was maintained over one month. Thus, the above immobilized enzyme membrane is stable for at least one month at 3°C.

Example C2

The immobilized Cellophane membrane of L-glutamic acid oxidase obtained according to the same procedure as in Example C1 was cut into a piece of circular shape, which was mounted on an electrode of a hydrogen peroxide electrode (produced by Ishikawa Seisakusho Co., Ltd.), and further the immobilized enzyme membrane was covered with a cellulose dialyzing membrane to prepare an L-glutamic acid sensor which has the similar structure as that of the sensor prepared in Example C1. By the use of the above sensor, an L-glutamic acid analyzing device similar to the device of Example C1 was prepared.

Sodium L-glutamate standard solutions were injected into the above device for measurement of the current values, from which the correlation between sodium L-glutamate concentrations and the current values was obtained as shown in Fig. 22.

Example C3

A commercially available soy sauce was diluted 10-fold with 0.1 M phosphate buffer (pH 7.0) and subjected to bubbling with air to saturate oxygen. The above sample solution (1 ml) was injected into 49 ml of 0.1 M phosphate buffer (pH 7.0) and assayed by means of the device prepared in Example C1. From the current reduction value obtained, with reference to the calibration curve, the L-glutamic acid concentration of the soy sauce was calculated. It was found that this value coincided well with the quantitatively determined value obtained by a commercially available L-glutamic acid autoanalyzer (produced by Technicon Instruments Co.) in which measurement is carried out by the colorimetric method using an L-glutamic acid decarboxylase.

Further, to a commercially available soy sauce was added a given amount (5—100 mM) of sodium L-glutamate for examination of recovery thereof with the use of the devices of Example C1 and Example C2. With reference to the calibration curve for each device, the amount of sodium L-glutamate recovered was calculated. It was found that the amount was substantially in proportion to the amount of sodium L-glutamate added as shown in Fig. 23 (the device of Example C1) and Fig. 24 (the device of Example C2).

**Claims**

1. An L-glutamic acid oxidase which is an L-amino acid oxidase having the activity of oxidative deamination of the α-amino group of L-glutamic acid in the presence of water and oxygen to form α-ketoglutaric acid, ammonia and hydrogen peroxide, having a very high substrate specificity for L-glutamic acid substantially without acting on L-glutamine, L-aspartic acid and L-histidine and having a high pH-stability and a high temperature stability characterized in that

a) it is stable in the pH range from pH 5.5 to pH 10.5 under the maintenance conditions of 37°C and 60 minutes;

b) has its optimum activity in the pH range from 7 to 8.5;

c) is not inactivated by cupric chloride in a concentration of 1.0 mM at pH 7.4 (see Table 2);

d) contains 2 mols of flavin adenine dinucleotide per mol of the enzyme as a coenzyme;

e) has a molecular weight of 135,000±10,000 as estimated by the gel filtration method;

f) is obtainable by culturing a microorganism of the species Streptomyces X-119-6 (FERM P-6560).

2. An L-glutamic acid oxidase as claimed in claim 1 characterized in that it is not inactivated by diethyldithiocarbamate in a concentration of 1.0 mM at pH 7.4.

3. A method for analysis of L-glutamic acid, which comprises causing the L-glutamic acid oxidase as claimed in claim 1 to react with L-glutamic acid in a sample to be analyzed in the presence of oxygen and water, and measuring the consumption of oxygen or the formation of hydrogen peroxide, of ammonia or of α-ketoglutaric acid accompanying the reaction.

4. A method according to claim 3, characterized in that the sample to be analyzed contains L-aspartic acid together with L-glutamic acid and that the method is carried out at a pH of preferably 5 to 6.

5. A reagent for analysis of L-glutamic acid comprising a buffering agent suitable for the enzyme reaction together with the L-glutamic acid oxidase as claimed in claim 1.

6. A reagent for analysis of L-glutamic acid according to claim 4 wherein the L-glutamic acid oxidase is immobilized on a carrier.

7. A kit for analysis of L-glutamic acid comprising an L-glutamic acid oxidase as claimed in claim 1 and a detecting reagent for the reaction by the enzyme.

8. A kit for analysis of L-glutamic acid according to claim 7 wherein the detecting reagent is a reagent for detection of hydrogen peroxide, ammonia or α-ketoglutaric acid.

9. A kit for analysis of L-glutamic acid according to any of claims 7 or 8 wherein the detecting reagent is a single color forming agent or a combined reagent of a color forming agent and a coupler.

10. A biosensor comprising a receptor portion for a substance to be detected comprising an enzyme and a transducer portion which detects the chemical or physical change in a sample for analysis by the action of said enzyme and transduces it to an electrical signal, characterized in that the L-glutamic acid oxidase which is claimed in claim 1 is used as said enzyme.

11. A biosensor according to claim 10 wherein the transducer portion is an electrode which detects a chemical change in the sample and transduces it to an electrical signal.

12. A biosensor according to claim 10 or claim 11 wherein the receptor portion comprises an immobilized enzyme.

**Patentansprüche**

1. Eine L-Glutaminsäureoxidase, welche eine L-Aminosäureoxidase ist, welche eine oxidative Desaminierung der α-Aminogruppe von L-Glutaminsäure in Gegenwart von Wasser und Sauerstoff zu α-Ketoglutarsäure, Ammoniak und Wasserstoffperoxid bewirkt, mit einer sehr hohen Substratespezifität für L-Glutaminsäure, ohne wesentlich auf L-Glutamin, L-Asparaginsäure und L-Histidin zu wirken und mit einer hohen pH-Stabilität und einer hohen Temperaturstabilität, dadurch gekennzeichnet, daß

a) sie im pH-Bereich von 5,5 bis 10,5 unter den Bedingungen von 37°C während 60 Minuten stabil ist;

b) sie die höchste Wirksamkeit im pH-Bereich von 7 bis 8,5 zeigt;

c) sie nicht durch Kupfer(II)chlorid in einer Konzentration von 1,0 mM bei einem pH-Wert von 7,4 desaktiviert wird(siehe Tabelle 2);

d) sie 2 Mol Flavinadenindinukleotid pro Mol des Enzyms als Coenzym enthält;

e) sie ein geschätztes Molekulargewicht von 135 000±10 000 hat, bestimmt mittels der Gelfiltrationsmethode;

f) sie durch Züchten eines Mikroorganismus der Spezies Streptomyces X-119-6 (FERM P-6560) erhältlich ist;

2. Eine L-Glutaminsäureoxidase, wie in Anspruch 1 beansprucht, dadurch gekennzeichnet, daß sie nicht durch Diäthyldithiocarbamat in einer Konzentration von 1,0 mM bei einem pH-Wert von 7,4 desaktiviert wird.

3. Ein Verfahren zur Analyse von L-Glutaminsäure, welches umfaßt, daß man die L-Glutaminsäureoxidase, wie in Anspruch 1 beansprucht, mit L-Glutaminsäure in einer Probe, welche zu analysieren ist in Gegenwart von Sauerstoff und Wasser reagieren läßt und den Verbrauch an Sauerstoff oder die Bildung von Wasserstoffperoxid, von Ammoniak oder von α-Ketoglutarsäure, welche Nebenprodukte der Reaktion sind, mißt.

4. Ein Verfahren nach Anspruch 3, dadurch gekennzeichnet, daß die zu analysierende Probe L-Asparaginsäure zusammen mit L-Glutaminsäure enthält und daß das Verfahren bei einem pH-Wert von vorzugsweise 5 bis 6 durchgeführt wird.

5. Ein Reagens zur Analyse von L-Glutaminsäure, umfassend eine Puffersubstanz, welche für die Enzymreaktion geeignet ist, zusammen mit der L-Glutaminsäureoxidase, wie in Anspruch 1 beansprucht.

6. Ein Reagens zur Analyse von L-Glutaminsäure nach Anspruch 4, in welcher die L-Glutaminsäureoxidase auf einem Träger immobilisiert vorliegt.

7. Eine Test-Ausrüstung zur Analyse von L-Glutaminsäure, enthaltend eine L-Glutaminsäureoxidase wie in Anspruch 1 beansprucht, und ein Nachweisreagens für die Reaktion durch das Enzym.

8. Eine Test-Ausrüstung zur Analyse von L-Glutaminsäure nach Anspruch 7, wobei das Nachweisreagens ein Reagens zum Nachweis von Wasserstoffperoxid, Ammoniak oder α-Ketoglutarsäure ist.

9. Eine Test-Ausrüstung zur Analyse von L-Glutaminsäure nach einem der Ansprüche 7 oder 8, wobei das Nachweisreagens ein einzelnes Färbemittel ist oder ein zusammengesetztes Reagens aus einem Färbemittel und einem Kupplungsmittel.

10. Ein Biosensor enthaltend einen Receptorteil für eine nachzuweisende Substanz, enthaltend ein Enzym und einen Umwandlungsteil, welcher die chemische oder physikalische Veränderung in einer zu analysierenden Probe infolge der Wirkung des genannten Enzyms nachweist und sie in ein elektrisches Signal umwandelt, dadurch gekennzeichnet, daß die L-Glutaminsäureoxidase, welche in Anspruch 1 beansprucht ist, als dieses Enzym eingesetzt wird.

11. Ein Biosensor nach Anspruch 10, in welchem der Umwandlungsteil eine Elektrode ist, welche eine chemische Veränderung in der Probe nachweist und sie in ein elektrisches Signal umwandelt.

12. Ein Biosensor nach Anspruch 10 oder 11, in welcher der Rezeptorteil ein immobilisiertes Enzym enthält.

**Revendications**

1. (Acide L-glutamique)oxydase qui est une (L-amino-acide)oxydase exerçant l'activité de désamination oxydative du groupe α-amino de l'acide L-glutamique en présence d'eau et d'oxygène en formant de l'acide α-céto-glutarique, de l'ammoniac et du peroxyde d'hydrogène, ayant une très grande spécificité de substrat envers l'acide L-glutamique sans agir sensiblement sur la L-glutamine, l'acide L-aspartique et le L-histidine et ayant une grande stabilité au pH et une grande stabilité à la température, caractérisée en ce que

a) elle est stable dans l'intervalle de pH de 5,5 à 10,5 dans les conditions d'un maintien à 37°C pendant 60 minutes;

b) son activité optimale se présente dans l'intervalle de pH de 7 à 8,5;

c) elle n'est pas inactivée par le chlorure cuivrique à une concentration 1,0 mM à pH 7,4 (voir tableau 2);

d) elle contient 2 moles de flavine-adénine-dinucléotide par mole de l'enzyme comme coenzyme;

e) son poids moléculaire est de 135 000±10 000, tel qu'estimé par la méthode de filtration sur gel;

f) elle peut être obtenue par culture d'un microorganisme de l'espèce Streptomyces X-119-6 (FERM P-6560).

2. (Acide L-glutamique)oxydase selon la revendication 1, caractérisée en ce qu'elle n'est pas inactivée par du diéthyldithiocarbamate à une concentration 1,0 mM à pH 7.4.

3. Méthode d'analyse pour la détermination de l'acide L'glutamique, qui consiste à faire réagir la (acide L-glutamique)oxydase telle que revendiquée dans la revendication 1 avec l'acide L-glutamique contenu dans un échantillon à analyser en présence d'oxygène et d'eau, et à mesurer la consommation d'oxygène ou la production de peroxyde d'hydrogène, d'ammoniac ou d'acide α-cétoglutarique accompagnant la réaction.

4. Méthode selon la revendication 3, caractérisée en ce que l'échantillon à analyser contient de l'acide

L-aspartique ainsi que de l'acide L-glutamique et en ce que la méthode est conduite à un pH de 5 à 6 de préférence.

5. Réactif d'analyse pour la détermination de l'acide L-glutamique, comprenant un agent tampon convenant pour la réaction enzymatique, ainsi que de la (acide L-glutamique)oxydase telle que revendiquée dans la revendication 1.

6. Réactif d'analyse pour la détermination de l'acide L'glutamique selon la revendication 4, dans lequel la (acide L-glutamique)oxydase est immobilisée sur un support.

7. Kit d'analyse pour la détermination de l'acide L-glutamique, comprenant une (acide L-glutamique)oxydase telle que revendiquée dans la revendication 1 et un réactif de détection pour détecter la réaction par l'enzyme.

8. Kit d'analyse pour la détermination de l'acide L-glutamique selon la revendication 7, dans lequel le réactif de détection est un réactif pour la détection du peroxyde d'hydrogène, de l'ammoniac ou de l'acide α-cétoglutarique.

9. Kit d'analyse pour la détermination de l'acide L-glutamique selon l'une quelconque des revendications 7 et 8, dans lequel le réactif de détection est un unique agent chromogène ou un réactif combiné consistant en un agent chromogène et un agent de copulation.

10. Biocapteur comprenant une partie réceptrice, comprenant une enzyme, pour une substance à détecter, et une partie transductrice qui détecte la modification chimique ou physique provoquée dans un échantillon à analyser par l'action de ladite enzyme et les traduit en un signal électrique, caractérisé en ce que la (acide L-glutamique)oxydase qui est revendiquée dans la revendication 1 est utilisée à titre de ladite enzyme.

11. Biocapteur selon la revendication 10, dans lequel la partie transductrice est une électrode qui détecte une modification chimique dans l'échantillon et la traduit en un signal électrique.

12. Biocapteur selon la revendication 10 ou la revendication 11, dans lequel la partie réceptrice consiste en une enzyme immobilisée.

FIG. 1

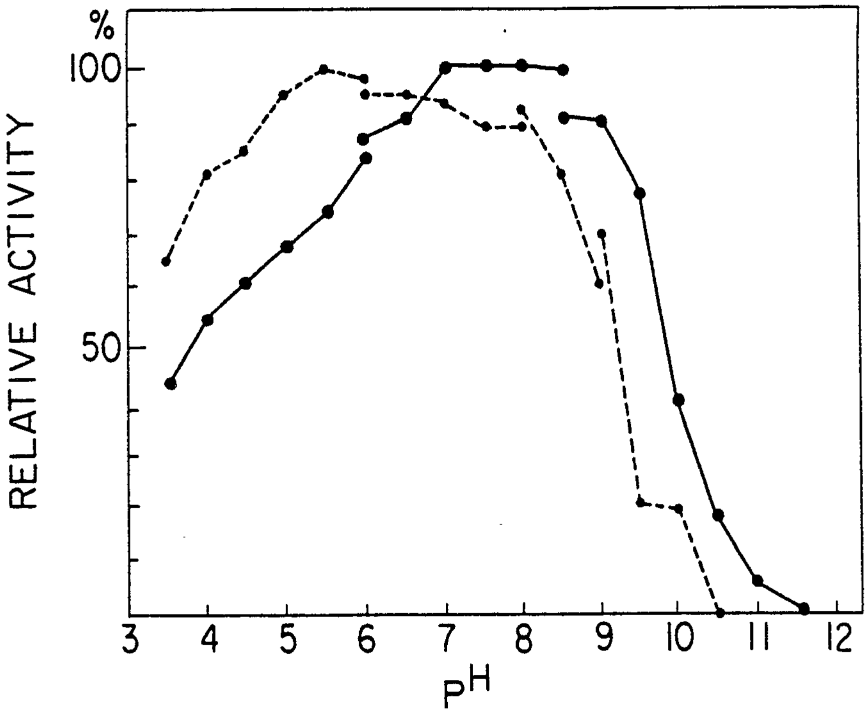
%
100
50
RELATIVE ACTIVITY
3
4
5
6
7
8
9
10
11
12
pH

FIG. 2

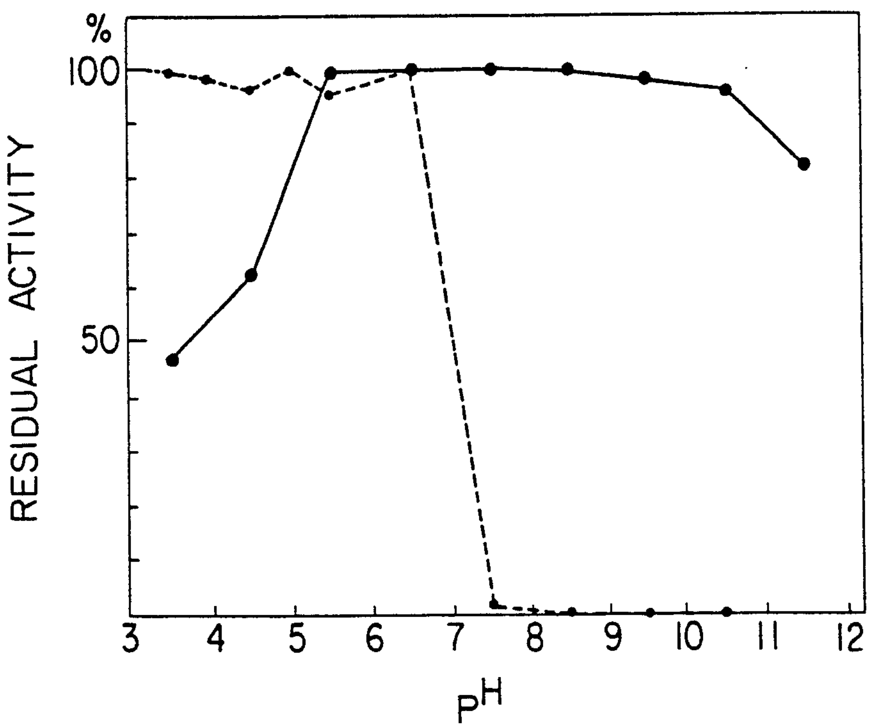
%
100
50
RESIDUAL ACTIVITY
3
4
5
6
7
8
9
10
11
12
pH

FIG. 3

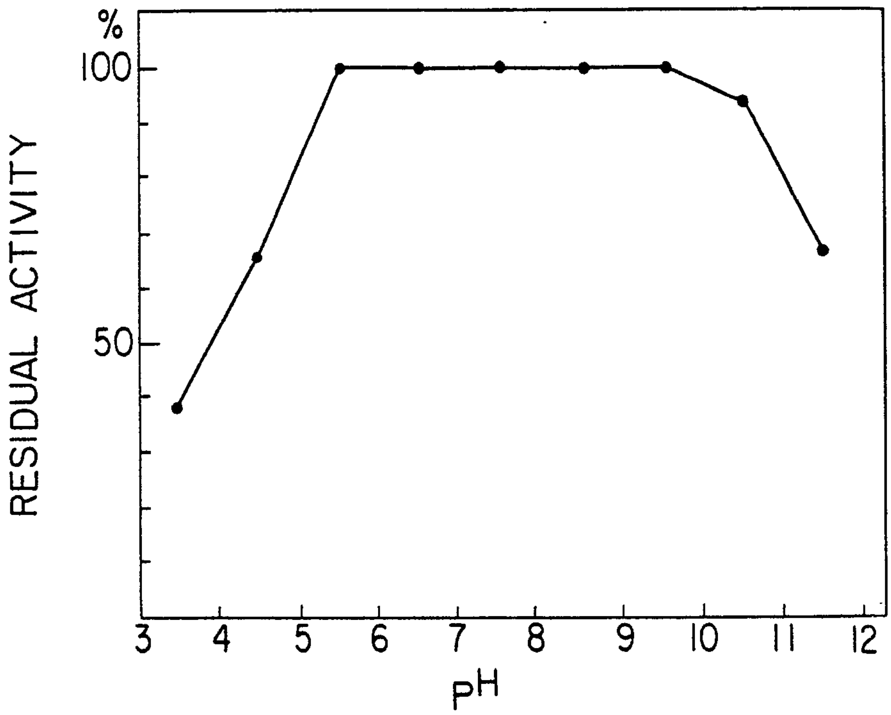
%
100
50
RESIDUAL ACTIVITY
3
4
5
6
7
8
9
10
11
12
pH

FIG. 4

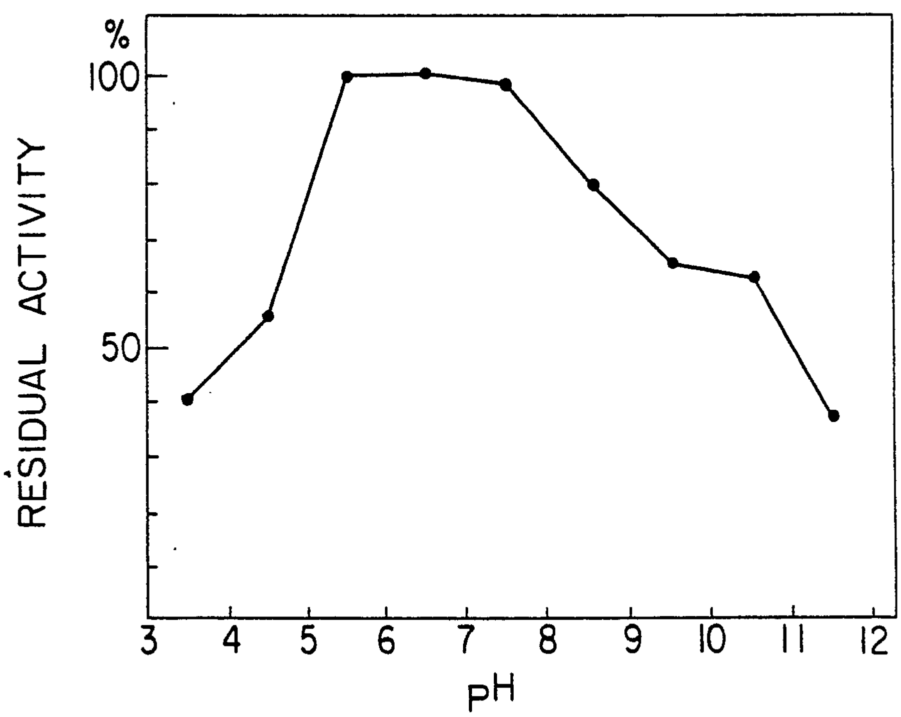
%
100
50
RESIDUAL ACTIVITY
3
4
5
6
7
8
9
10
11
12
pH

FIG. 5

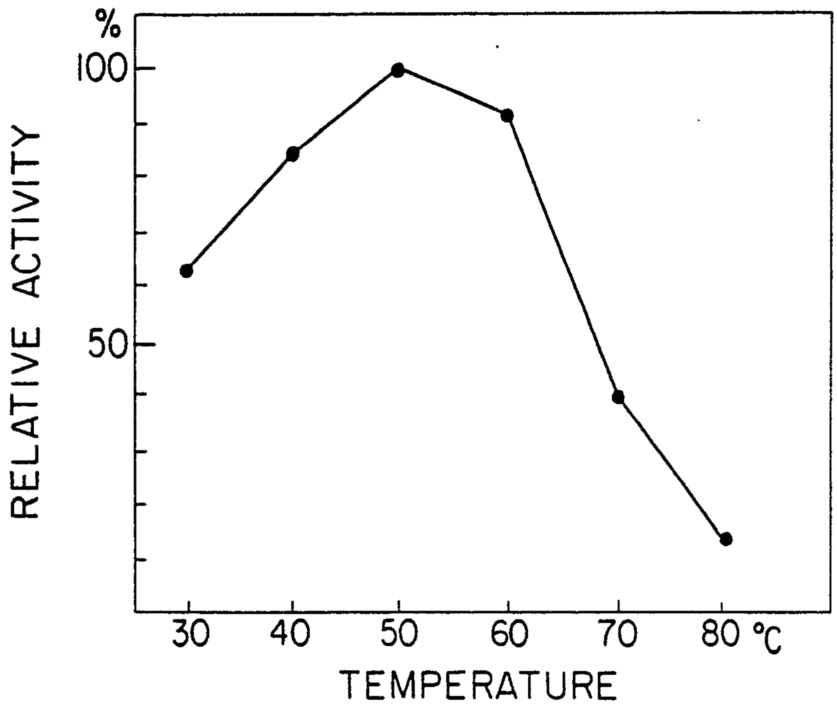
%
100
50
RELATIVE ACTIVITY
30
40
50
60
70
80 °C
TEMPERATURE

FIG. 6

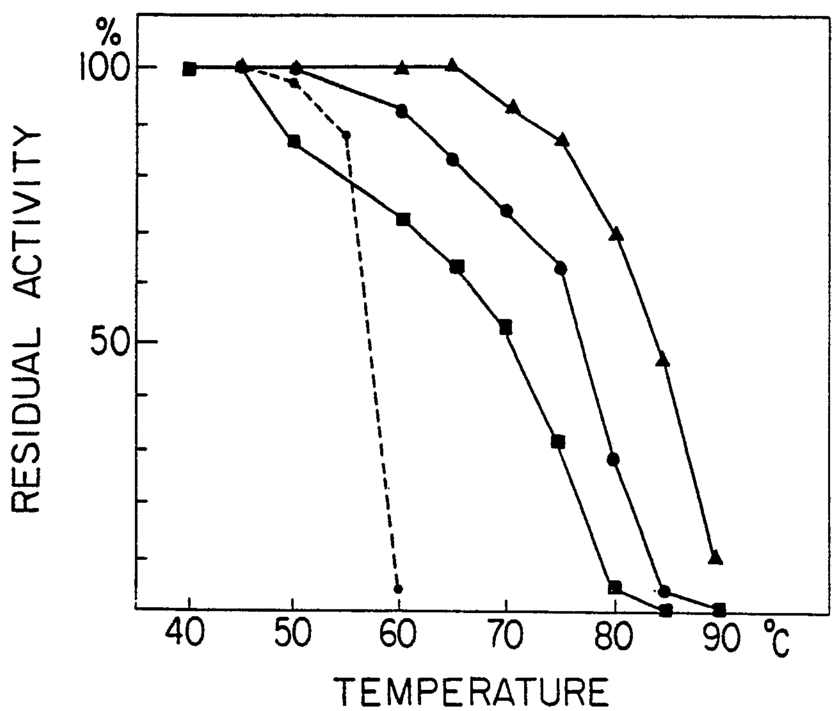
%
100
50
RESIDUAL ACTIVITY
40
50
60
70
80
90 °C
TEMPERATURE

FIG. 7

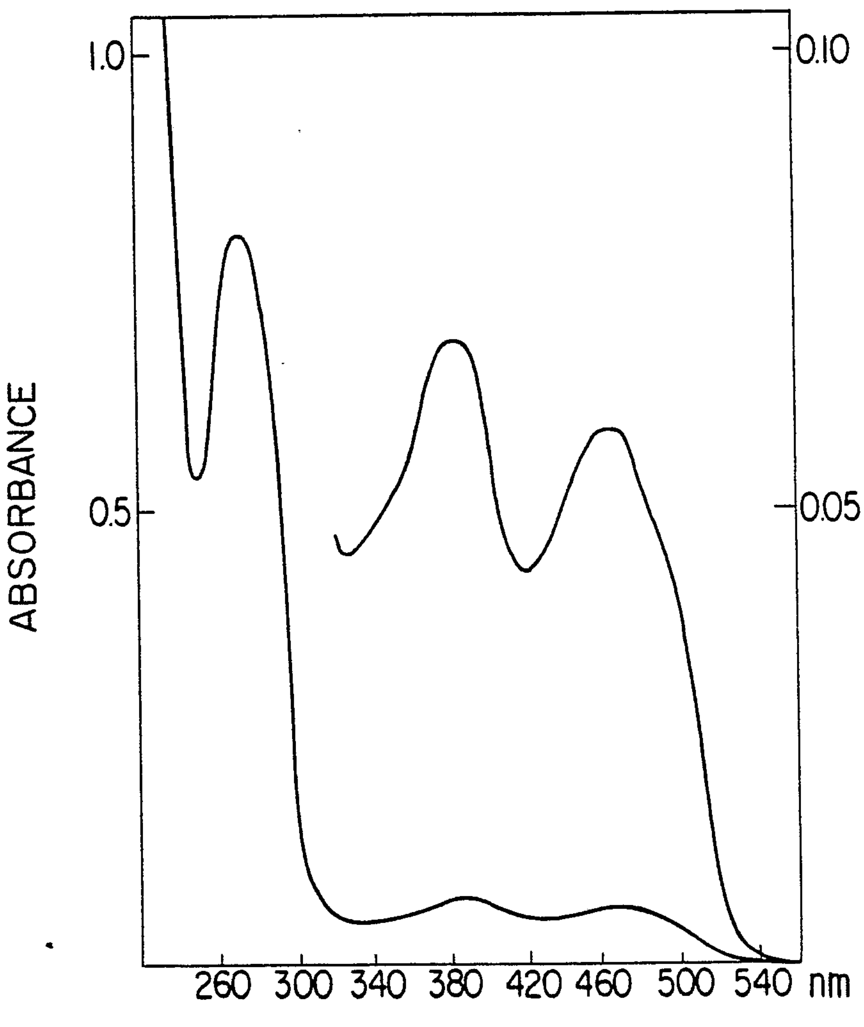

FIG. 8

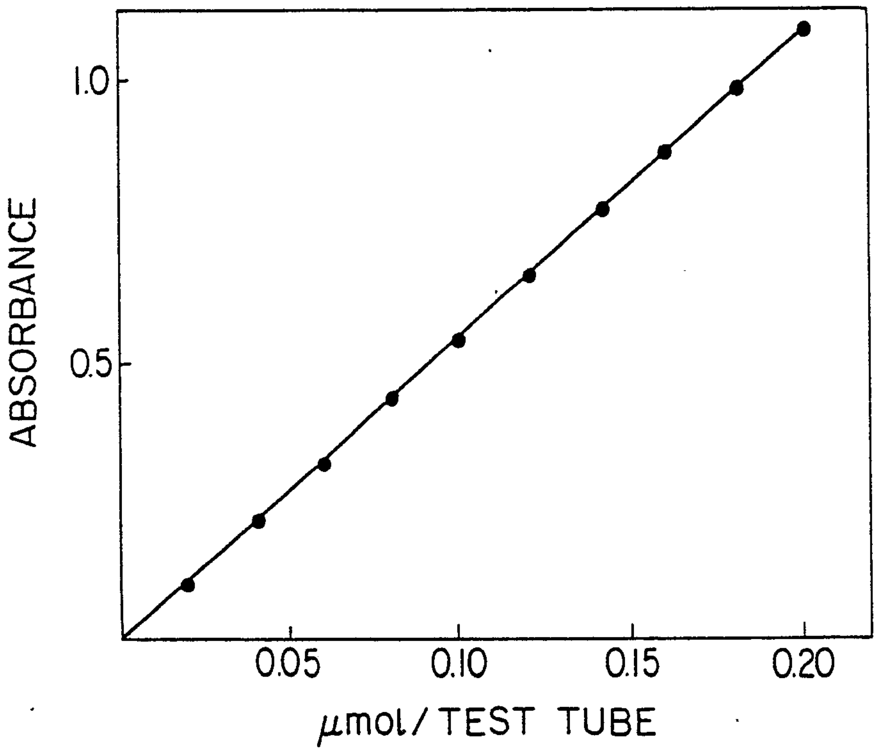
1.0
0.5
ABSORBANCE
0.05
0.10
0.15
0.20
μmol/TEST TUBE

FIG. 9

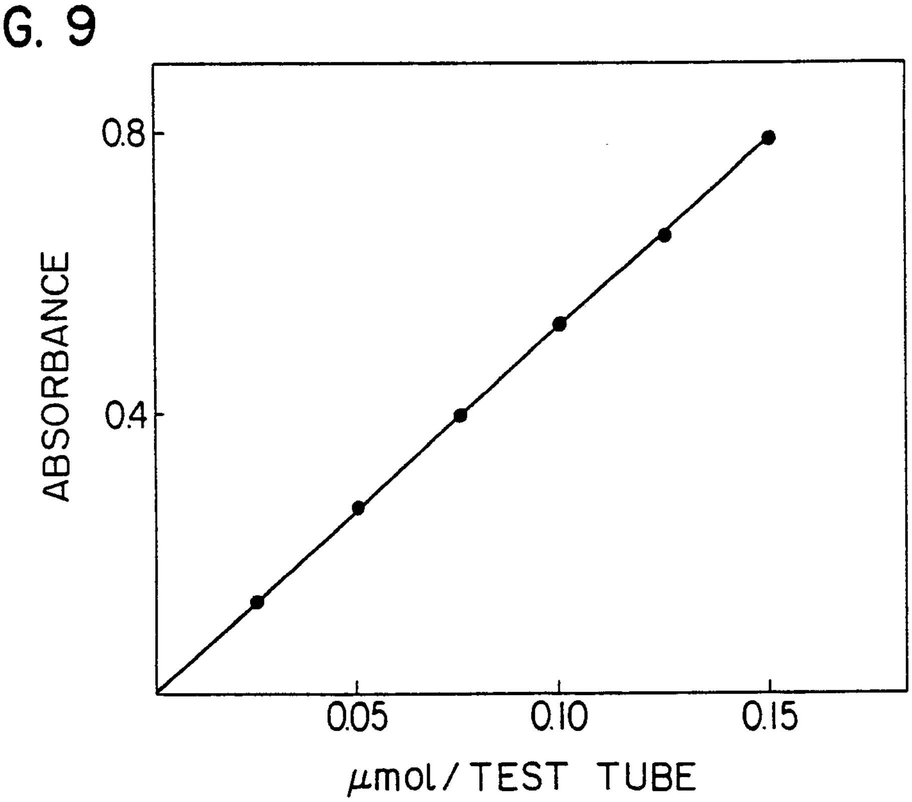
0.8
0.4
ABSORBANCE
0.05
0.10
0.15
μmol/TEST TUBE

FIG. 10

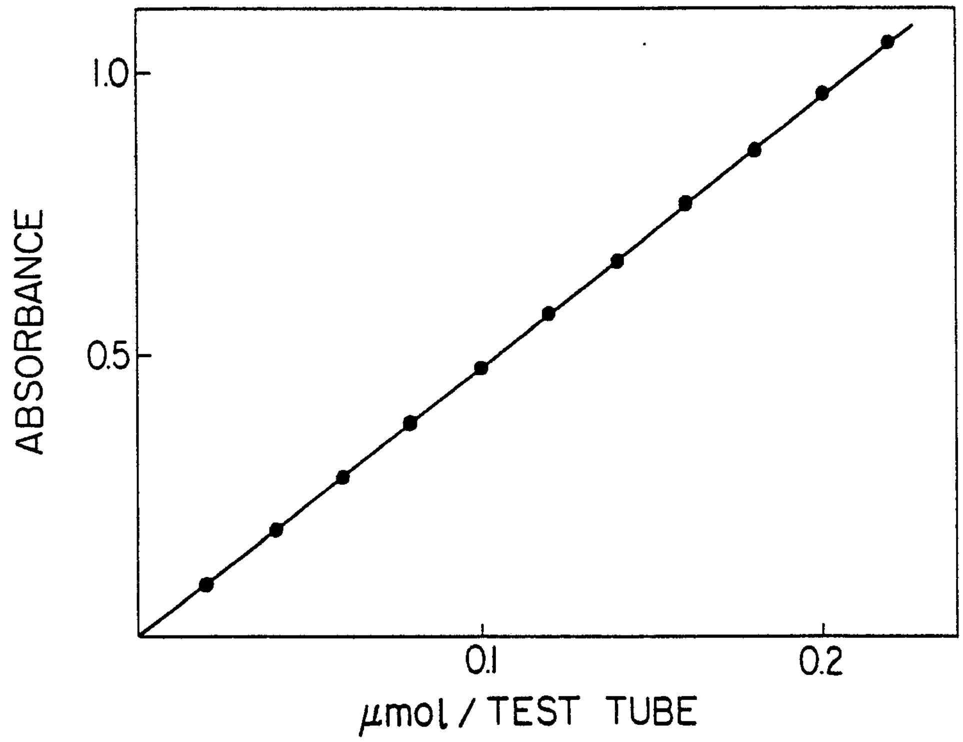
ABSORBANCE
1.0
0.5
0.1
0.2
μmol / TEST TUBE

FIG. 11

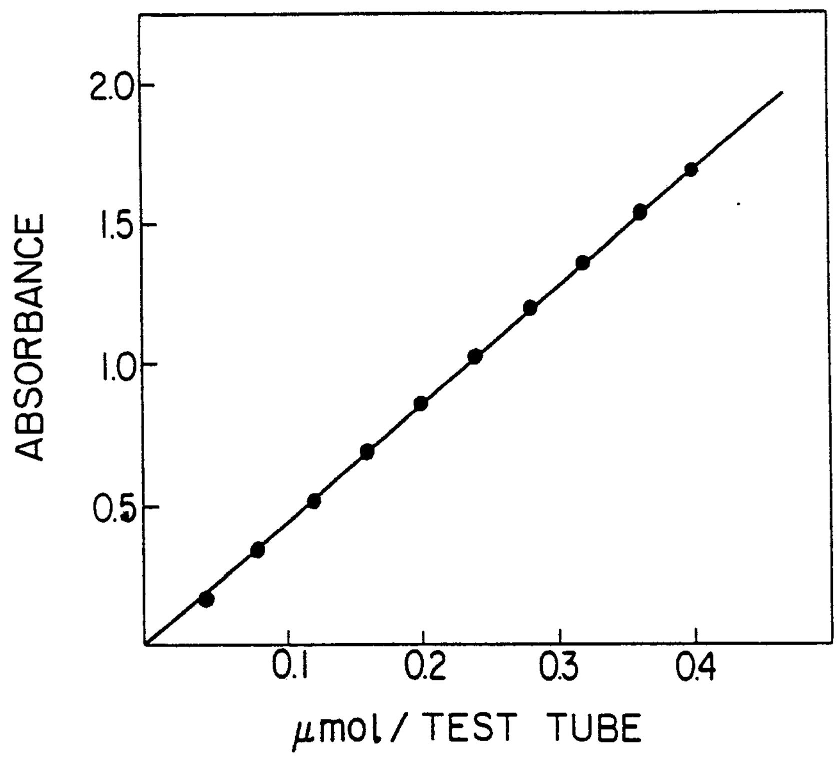
ABSORBANCE
2.0
1.5
1.0
0.5
0.1
0.2
0.3
0.4
μmol / TEST TUBE

FIG. 12

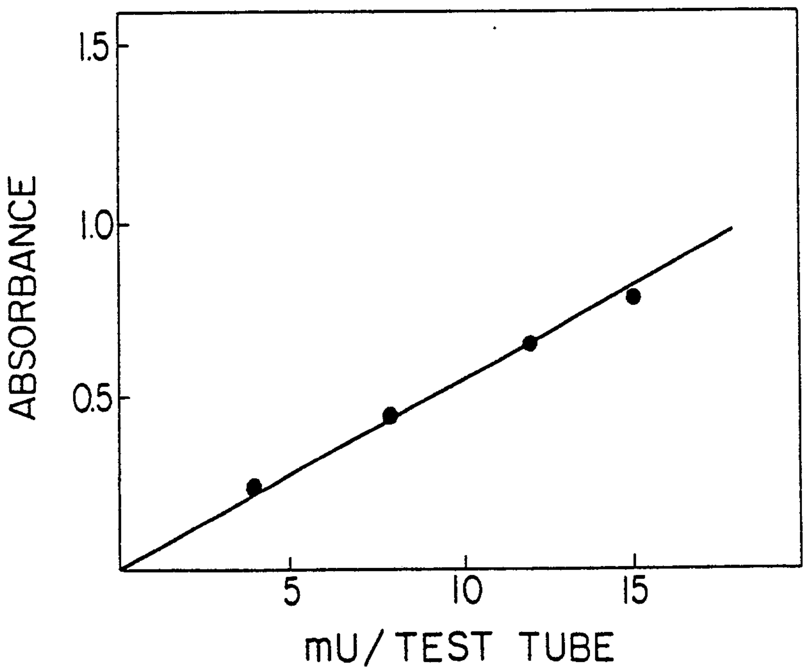
1.5
1.0
0.5
ABSORBANCE
5
10
15
mU/ TEST TUBE

FIG. 13

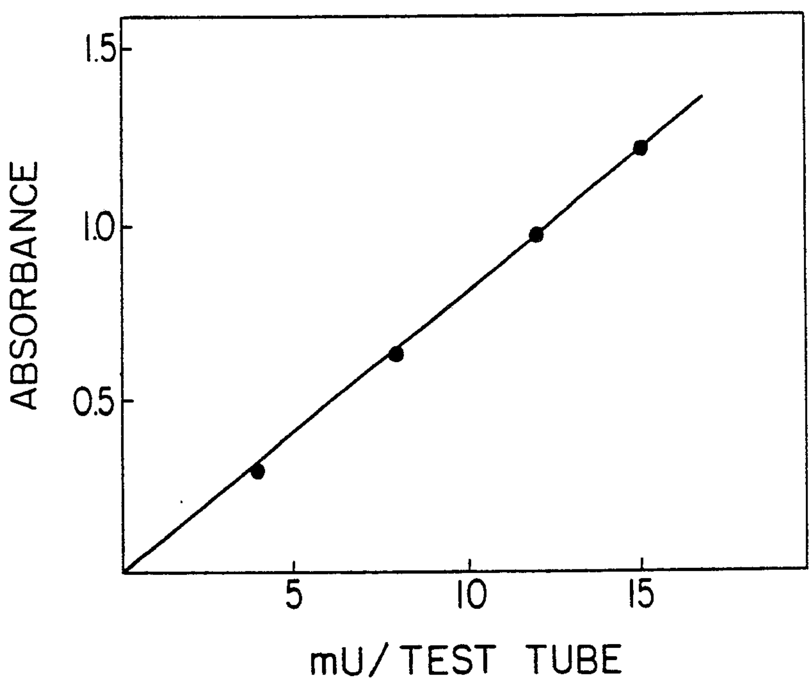
1.5
1.0
0.5
ABSORBANCE
5
10
15
mU/ TEST TUBE

FIG. 14

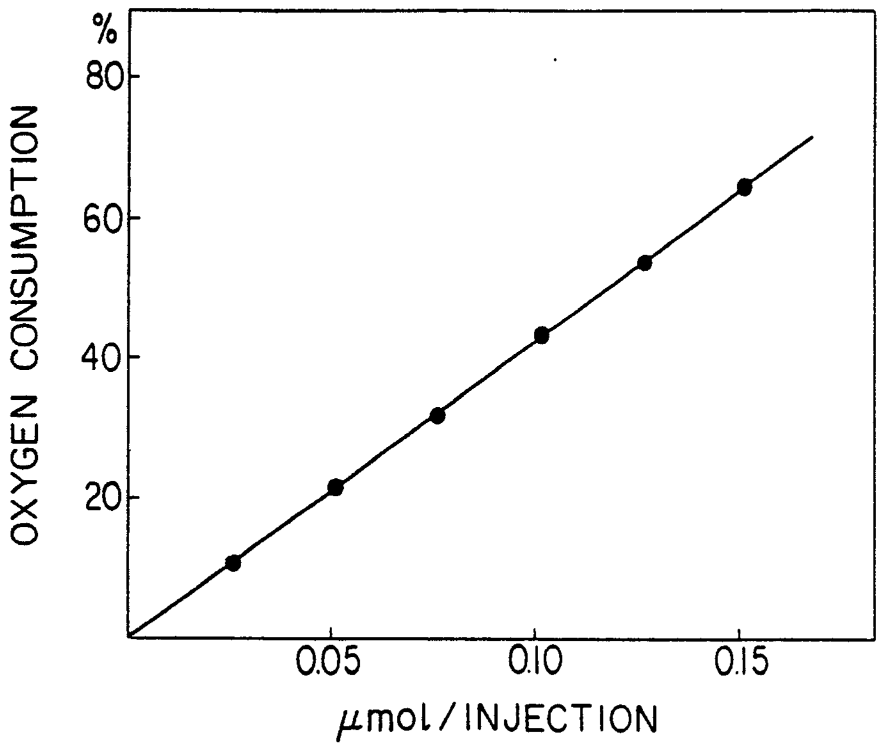
%
80
60
40
20
OXYGEN CONSUMPTION
0.05
0.10
0.15
μmol/INJECTION

FIG. 15

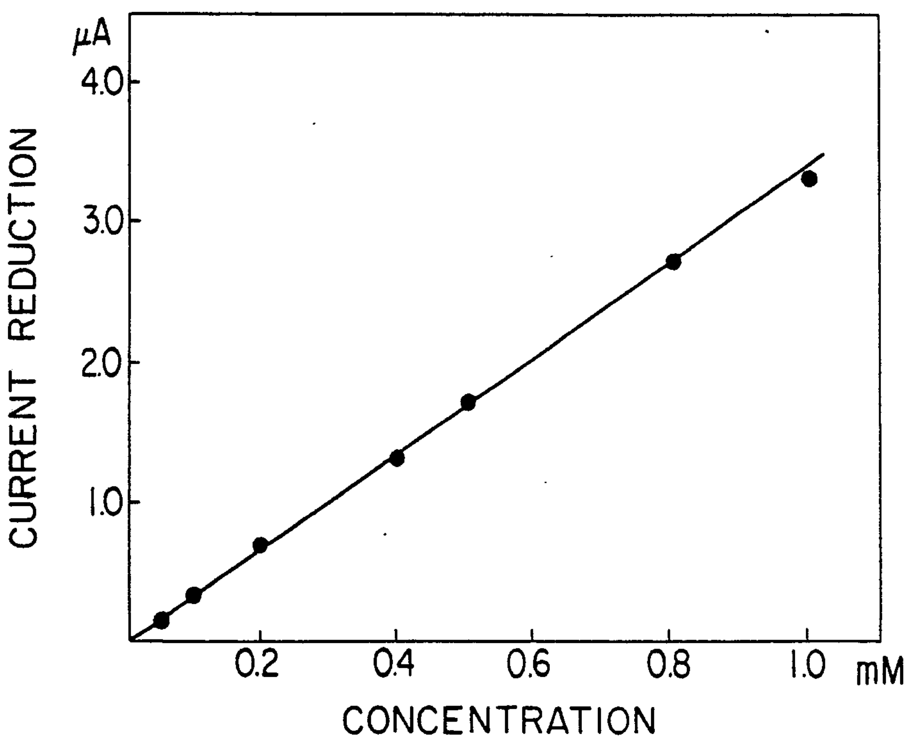
μA
4.0
3.0
2.0
1.0
CURRENT REDUCTION
0.2
0.4
0.6
0.8
1.0
mM
CONCENTRATION

FIG. 16

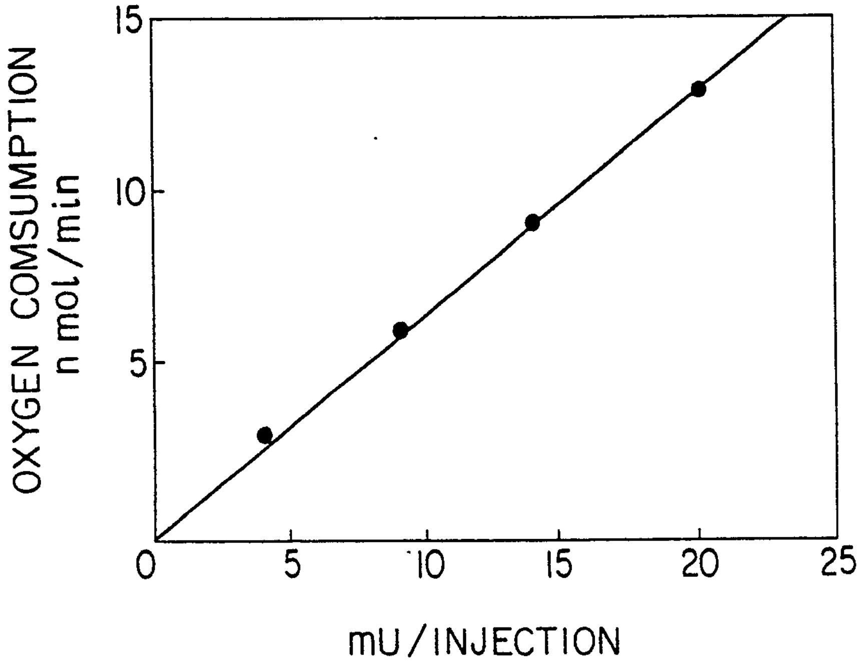
OXYGEN COMSUMPTION
n mol/min
15
10
5
0
5
10
15
20
25
mU/INJECTION

FIG. 17

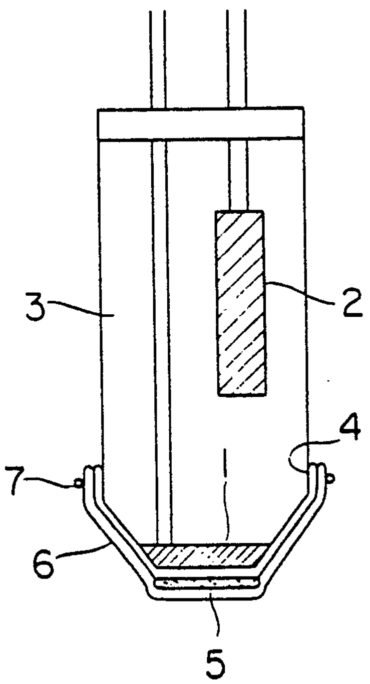


FIG. 18

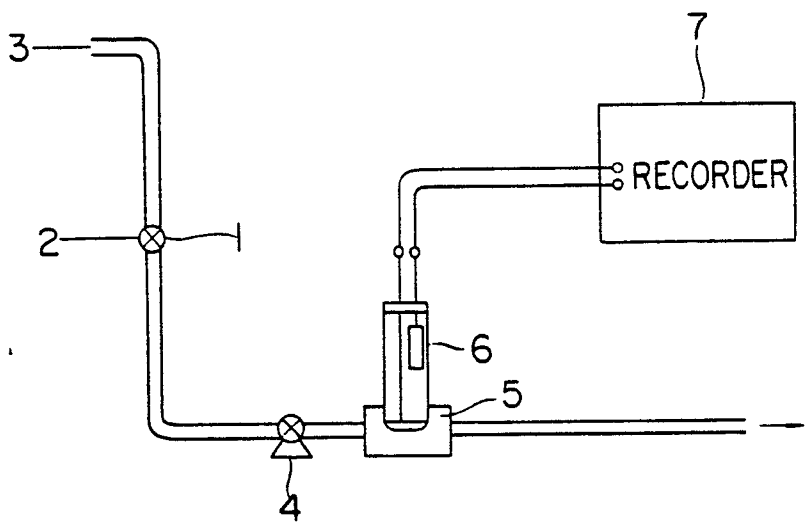

FIG. 19

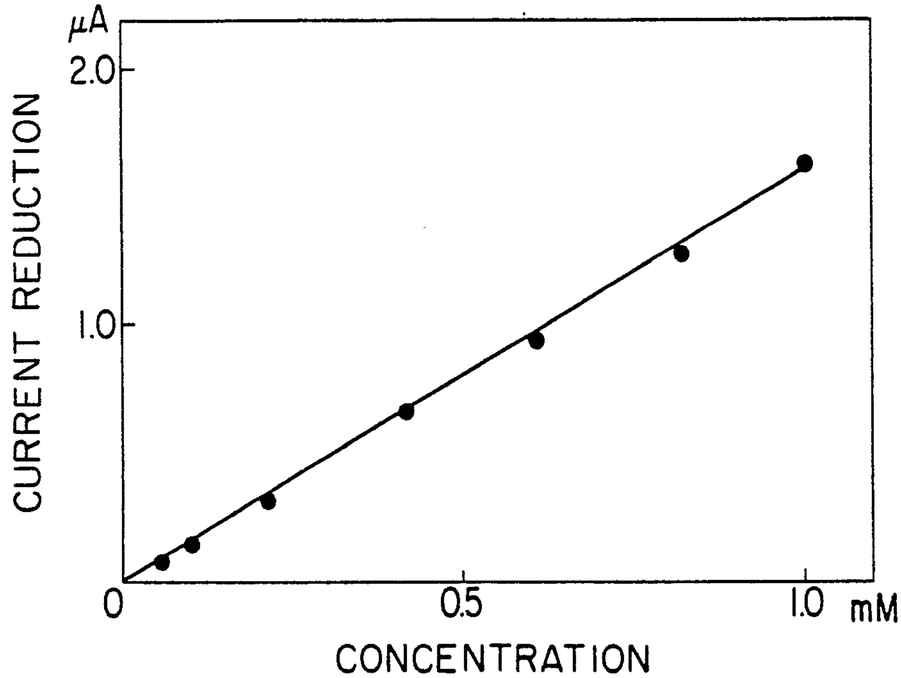
μA
2.0
1.0
CURRENT REDUCTION
0
0.5
1.0
mM
CONCENTRATION

FIG. 20

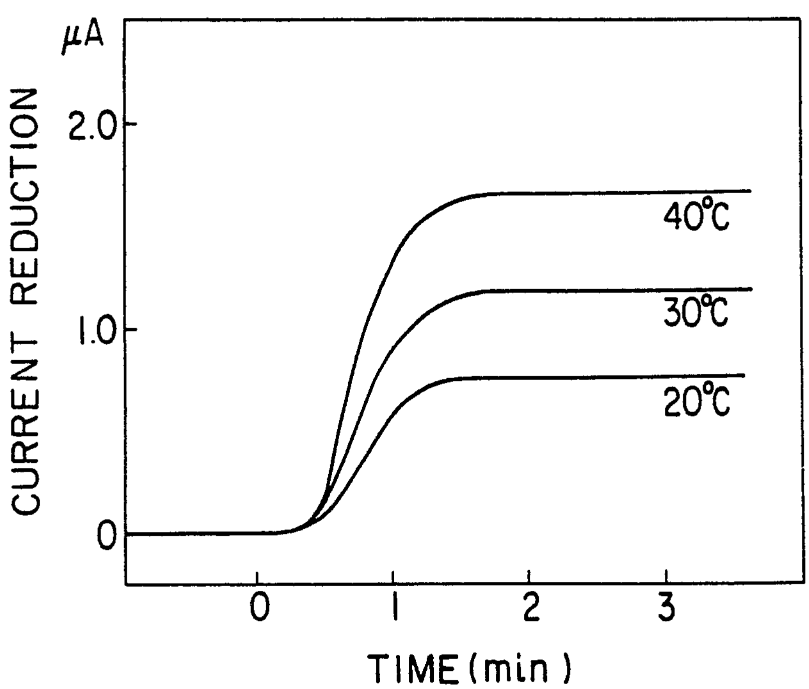
μA
2.0
1.0
0
CURRENT REDUCTION
40°C
30°C
20°C
0
1
2
3
TIME (min)

FIG. 21

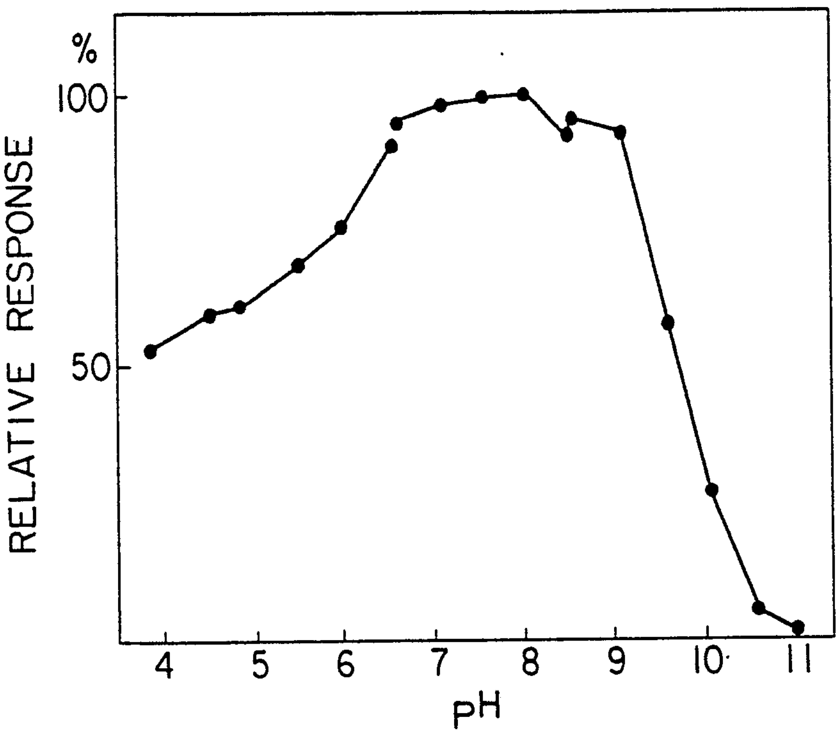
%
100
50
RELATIVE RESPONSE
4
5
6
7
8
9
10
11
pH

FIG. 22

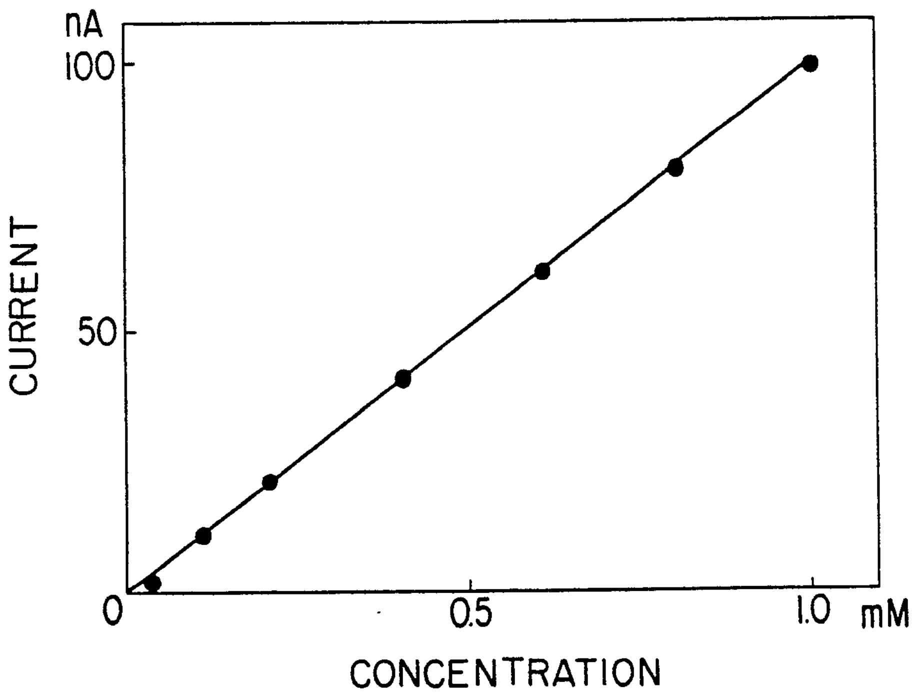
nA
100
50
CURRENT
0
0.5
1.0 mM
CONCENTRATION

FIG. 23

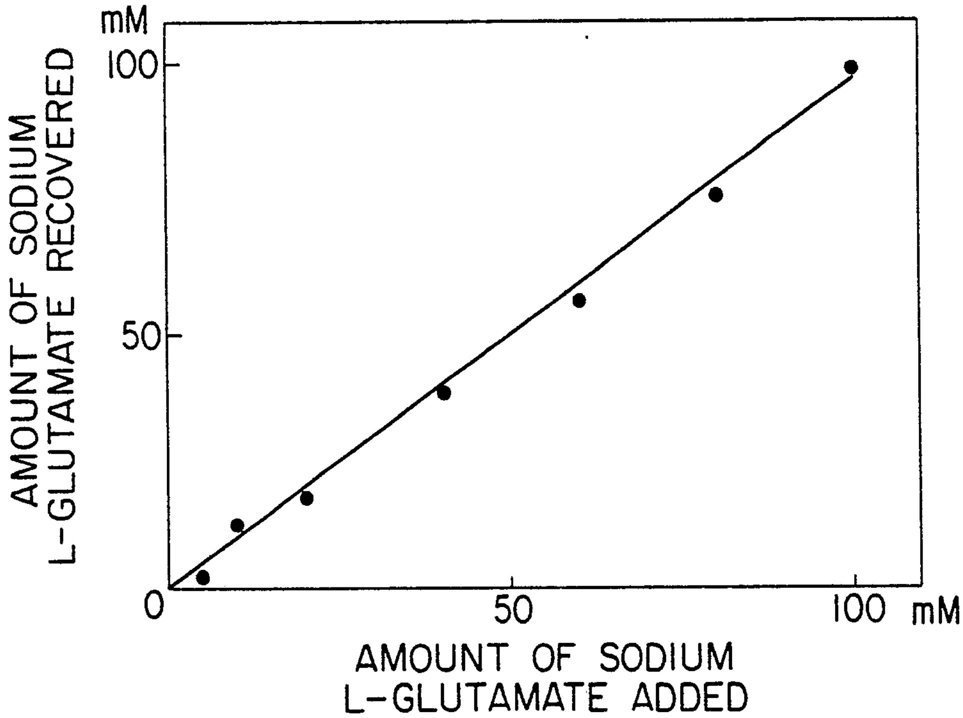
mM
100
50
0
AMOUNT OF SODIUM
L-GLUTAMATE RECOVERED
50
100 mM
AMOUNT OF SODIUM
L-GLUTAMATE ADDED

FIG. 24

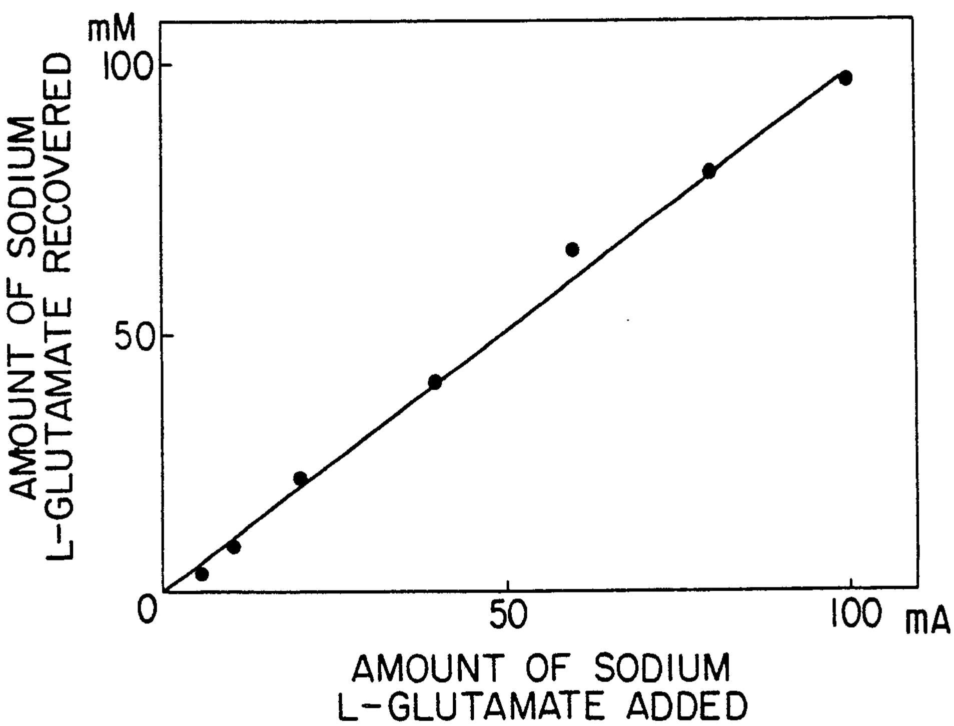
mM
100
50
0
AMOUNT OF SODIUM
L-GLUTAMATE RECOVERED
50
100 mA
AMOUNT OF SODIUM
L-GLUTAMATE ADDED